(19) **Europäisches Patentamt / European Patent Office / Office européen des brevets**

(11) **EP 4 256 345 B1**

(12) **EUROPEAN PATENT SPECIFICATION**

(45) Date of publication and mention of the grant of the patent:
**04.03.2026 Bulletin 2026/10**

(21) Application number: **21836692.0**

(22) Date of filing: **03.12.2021**

(51) International Patent Classification (IPC):
**G01N 33/58** (2006.01)    **G01N 33/543** (2006.01)
**G16H 50/20** (2018.01)    **G01N 33/68** (2006.01)

(52) Cooperative Patent Classification (CPC):
**G01N 33/582; G01N 33/543; G01N 33/6896; G16H 10/40; G16H 10/60; G16H 50/20; G16H 50/30; G16H 50/70;** G01N 2333/4737; G01N 2800/30

(86) International application number:
**PCT/US2021/061803**

(87) International publication number:
**WO 2022/120164 (09.06.2022 Gazette 2022/23)**

(54) **METHODS FOR DYNAMIC IMMUNOHISTOCHEMISTRY PROFILING OF AUTISM SPECTRUM DISORDER**

VERFAHREN ZUR DYNAMISCHEN IMMUNHISTOCHEMIEPROFILIERUNG DER AUTISMUS-SPEKTRUM-STÖRUNG

PROFILAGE DYNAMIQUE D'IMMUNO-HISTOCHIMIE DES TROUBLES DU SPECTRE AUTISTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.12.2020 US 202063121792 P**

(43) Date of publication of application:
**11.10.2023 Bulletin 2023/41**

(73) Proprietor: **Icahn School of Medicine at Mount Sinai**
**New York, NY 10029 (US)**

(72) Inventors:
• **ARORA, Manish**
  **New York, NY 10029 (US)**
• **CURTIN, Paul**
  **New York, NY 10029 (US)**
• **AUSTIN, Christine**
  **New York, NY 10029 (US)**

(74) Representative: **Grund, Martin et al**
**Grund Intellectual Property Group**
**Patentanwälte und Solicitor PartG mbB**
**Steinsdorfstraße 2**
**80538 München (DE)**

(56) References cited:
• **ANON: "Visualising dental caries using fluorescence lifetime microscopy", TIME-RESOLVED FLUORESCENCE APPLICATION NOTE TRFA-3, 6 April 2016 (2016-04-06), XP55884387, Retrieved from the Internet <URL:https://web.archive.org/web/20160406181557if_/http://www.horiba.com/fileadmin/uploads/Scientific/Documents/Fluorescence/Appl_Note3_-_Dental_monitoring.pdf> [retrieved on 20220127]**

**(Cont. next page)**

- PUDNEY PAUL D ET AL: "Confocal Raman Spectroscopy of Whole Hairs", APPLIED SPECTROSCOPY, 1 December 2013 (2013-12-01), XP55885959, Retrieved from the Internet <URL:https://www.researchgate.net/profile/Paul-Pudney/publication/259446389_Confocal_Raman_Spectroscopy_of_Whole_Hairs/links/0046352d5723b5c897000000/Confocal-Raman-Spectroscopy-of-Whole-Hairs.pdf> [retrieved on 20220201], DOI: 10.1366/13-07086.Source:
- AUSTIN CHRISTINE ET AL: "Uncovering system-specific stress signatures in primate teeth with multimodal imaging", SCIENTIFIC REPORTS, vol. 6, no. 1, 1 May 2016 (2016-05-01), XP55884415, Retrieved from the Internet <URL:https://www.nature.com/articles/srep18802.pdf> DOI: 10.1038/srep18802
- MA LI-NA ET AL: "Assessment of high-sensitivity C-reactive protein tests for the diagnosis of hepatocellular carcinoma in patients with hepatitis B-associated liver cirrhosis", ONCOLOGY LETTERS, vol. 13, no. 5, 1 May 2017 (2017-05-01), GR, pages 3457 - 3464, XP55884325, ISSN: 1792-1074, Retrieved from the Internet <URL:https://www.ncbi.nlm.nih.gov/pmc/articles/PMC5431324/pdf/ol-13-05-3457.pdf> DOI: 10.3892/ol.2017.5890
- AUSTIN CHRISTINE ET AL: "Uncovering system-specific stress signatures in primate teeth with multimodal imaging", SCIENTIFIC REPORTS, vol. 6, no. 1, 1 May 2016 (2016-05-01), XP055884415, Retrieved from the Internet <URL:https://www.nature.com/articles/srep18802.pdf> DOI: 10.1038/srep18802
- MORISHITA HIROFUMI ET AL: "Tooth-Matrix Biomarkers to Reconstruct Critical Periods of Brain Plasticity", TRENDS IN NEUROSCIENCES, vol. 40, no. 1, January 2017 (2017-01-01), pages 1 - 3, XP029873898, ISSN: 0166-2236, DOI: 10.1016/J.TINS.2016.11.003
- ANON: "Visualising dental caries using fluorescence lifetime microscopy", TIME-RESOLVED FLUORESCENCE APPLICATION NOTE TRFA-3, 6 April 2016 (2016-04-06), XP055884387, Retrieved from the Internet <URL:https://web.archive.org/web/20160406181557if_/http://www.horiba.com/fileadmin/uploads/Scientific/Documents/Fluorescence/Appl_Note3_-_Dental_monitoring.pdf> [retrieved on 20220127]
- MIHAI R GHERASE ET AL: "X-ray fluorescence measurements of arsenic micro-distribution in human nail clippings using synchrotron radiation", PHYSIOLOGICAL MEASUREMENT, INSTITUTE OF PHYSICS PUBLISHING, BRISTOL, GB, vol. 34, no. 9, 23 August 2013 (2013-08-23), pages 1163 - 1177, XP020250055, ISSN: 0967-3334, [retrieved on 20130823], DOI: 10.1088/0967-3334/34/9/1163
- MAEKAWA MOTOKO ET AL: "Utility of Scalp Hair Follicles as a Novel Source of Biomarker Genes for Psychiatric Illnesses", BIOLOGICAL PSYCHIATRY, ELSEVIER, AMSTERDAM, NL, vol. 78, no. 2, 11 September 2014 (2014-09-11), pages 116 - 125, XP029215883, ISSN: 0006-3223, DOI: 10.1016/J.BIOPSYCH.2014.07.025
- LOPES NAIR ET AL: "Digital image analysis of multiplex fluorescence IHC in colorectal cancer recognizes the prognostic value of CDX2 and its negative correlation with SOX2", LABORATORY INVESTIGATION, NATURE PUBLISHING GROUP, THE UNITED STATES AND CANADIAN ACADEMY OF PATHOLOGY, INC, vol. 100, no. 1, 22 October 2019 (2019-10-22), pages 120 - 134, XP036966490, ISSN: 0023-6837, [retrieved on 20191022], DOI: 10.1038/S41374-019-0336-4
- MA LI-NA ET AL: "Assessment of high-sensitivity C-reactive protein tests for the diagnosis of hepatocellular carcinoma in patients with hepatitis B-associated liver cirrhosis", ONCOLOGY LETTERS, vol. 13, no. 5, 1 May 2017 (2017-05-01), GR, pages 3457 - 3464, XP055884325, ISSN: 1792-1074, Retrieved from the Internet <URL:https://www.ncbi.nlm.nih.gov/pmc/articles/PMC5431324/pdf/ol-13-05-3457.pdf> DOI: 10.3892/ol.2017.5890

**Description**

**BACKGROUND**

Dynamic biological responses may be indicative of underlying biological processes having structural and functional significance for humans. For example, aberrant or abnormal dynamic biological response may be associated with many biological conditions, such as diseases and disorders. Examples of such biological conditions may include neurological conditions (e.g., autism spectrum disorder, schizophrenia, or attention-deficit/hyperactivity disorder (ADHD)), neurodegenerative conditions (e.g., amyotrophic lateral sclerosis (ALS), Alzheimer's disease, Parkinson's disease, and Huntington's disease), and cancers (e.g., pediatric cancer).

Early life stress can disrupt development and negatively impact long-term health trajectories. It has been shown that external physical and social stressors impact biochemical signatures in primate teeth that can be retrieved to objectively reconstruct the timing of early life developmental disruptions. It was found that discrete increases in heat shock protein-70 expression in dentine coincided with elemental signatures, confirming that elemental signals were associated with activation of stress-related pathways. The structural and compositional alteration in enamel and dentine were analyzed using Raman microspectral imaging (see, Austin C. et al. (2016), SCIENTIFIC REPORTS, 6:18802). Moreover, monitoring tooth biomarkers with multiplexed mass-spectrometry has been shown to enable measuring changes in brain plasticity (see, Morishita H. et al. (2016), TRENDS IN NEUROSCIENCES, 40(1):1-3).

However, improved methods for predicting a subject's diagnostic status with respect to a disease or disorder comprising autism spectral disorder (ASD) are needed.

**SUMMARY**

The invention is defined by the appended claims.

The present invention relates to a method for predicting a subject's diagnostic status with respect to a disease or disorder, comprising:

(a) obtaining by imaging a fluorescence image of a stained tooth sample of the subject, wherein the stained tooth sample is stained using an immunohistochemistry stain;
(b) analyzing the stain fluorescence intensity of the fluorescence image spatially across the stained tooth sample; and
(c) predicting the subject's diagnostic status with respect to the disease or disorder based at least in part on the analysis of the fluorescence intensity, wherein the disease or disorder comprises autism spectrum disorder (ASD);

wherein staining the tooth sample comprises using a C-reactive protein immunohistochemistry stain; and wherein the subject is human and less than 12 years old.

In an embodiment, obtaining the fluorescence image of the stained tooth sample comprises using an inverted or non-inverted confocal microscope.

In an embodiment, the method may comprise sectioning the tooth sample.

In an embodiment, staining the tooth sample comprises decalcifying the tooth sample.

In a preferred embodiment, the subject is less than 1 year old.

Analyzing may comprise generating a temporal profile of inflammation based at least in part on the fluorescence intensity, and analyzing the temporal profile of inflammation.

In another embodiment, at least a portion of the temporal profile of inflammation corresponds to a prenatal period of the subject.

Predicting the subject's diagnostic status with respect to the disease or disorder may comprise processing the fluorescence intensity using a trained model. In such embodiment, the trained model may be selected from the group consisting of: a neural network algorithm, a support vector machine algorithm, a decision tree algorithm, an unsupervised clustering algorithm, a supervised clustering algorithm, a regression algorithm, a gradient-boosting algorithm, and any combination thereof, or the trained model may comprise a gradient-boosted decision tree. In such embodiment, the trained model may be configured to process one or more features selected from the group consisting of recurrence rates, determinism, mean diagonal length, maximum diagonal length, divergence, Shannon entropy in diagonal length, trend in recurrences, laminarity, trapping time, maximum vertical line length, Shannon entropy in vertical line lengths, mean recurrence time, Shannon entropy in recurrence times, number of the most probable recurrences, mean diagonal length (MDL), recurrence time (RT), Vmax, determinism, Lmax, and any combination thereof. In a further embodiment, the trained model may be configured to process two or more features selected from the group consisting of recurrence rates, determinism, mean diagonal length, maximum diagonal length, divergence, Shannon entropy in diagonal length, trend in recurrences, laminarity, trapping time (TT), maximum vertical line length, Shannon entropy in vertical line lengths, mean recurrence time, Shannon entropy in recurrence times, number of the most probable recurrences, mean diagonal length (MDL), recurrence time (RT), Vmax, determinism, Lmax, and any combination thereof. In a further embodiment, the trained model may predict the subject's diagnostic status with respect to the disease or disorder with a sensitivity of at least about 80%, or the trained model may predict the subject's diagnostic status with respect to the disease or disorder with a specificity of at least about 80%, or the trained model may predict the subject's diagnostic status with respect to the disease or disorder with a positive predictive value of at least about 80%, or the trained model may predict the subject's diagnostic status with respect to the disease or disorder with a negative predictive value of at least about 80%, or the trained model may predict the subject's diagnostic status with respect to the disease or disorder with an Area Under the Receiver Operating Characteristic (AUROC) of at least about 0.80.

## BRIEF DESCRIPTION OF THE DRAWINGS

FIG. 1 shows an example of a block diagram of a computing device 100 of the present disclosure.
FIGS. 2A-2C show illustrations of a hair sample (FIG. 2A), a tooth sample (FIG. 2B), and a nail sample (FIG. 2C) of a subject.
FIG. 3 shows a flow chart of a method 300 for evaluating a subject for a biological condition.
FIG. 4 shows a computer system that is programmed or otherwise configured to implement methods provided herein.
FIG. 5 shows an example of a daily C-reactive protein profile of a subject over time, where the y-axis is indicative of CRP intensity and the x-axis is indicative of developmental age.

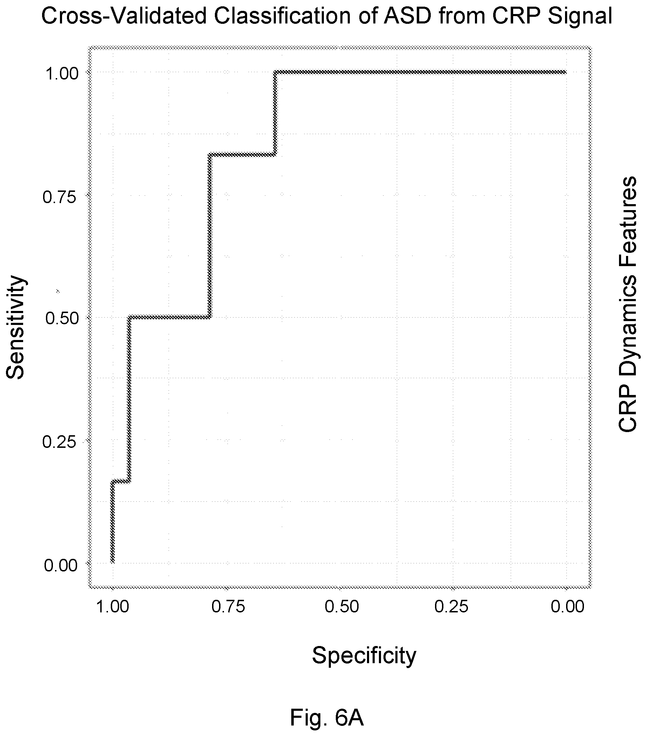

FIGS. 6A-6B show a receiver operating characteristic (ROC) curve to characterize the sensitivity and specificity of the method for diagnosing autism at varying predictive thresholds with a model trained utilizing features derived from recurrence quantification analysis of C-reactive protein profiles sampled prenatally and in early childhood (e.g., up to 1 year of age). Device performance is measured by calculating the area-under-the-curve (AUC) of the ROC plot, which provides a measure of performance at varying classification thresholds; here, the AUC was 0.86, indicating robustly accurate predictive performance.

## DETAILED DESCRIPTION

Dynamic biological responses may be indicative of underlying biological processes having structural and functional significance for humans. For example, aberrant or abnormal dynamic biological response may be associated with many biological conditions, such as diseases and disorders. Examples of such biological conditions may include neurological conditions (e.g., autism spectrum disorder, schizophrenia, or attention-deficit/hyperactivity disorder (ADHD)), neurodegenerative conditions (e.g., amyotrophic lateral sclerosis (ALS), Alzheimer's disease, Parkinson's disease, and Huntington's disease), and cancers (e.g., pediatric cancer).

Given the above background, there is a need for accurate methods and systems for the diagnosis of biological conditions, and especially for non-invasive diagnosis. Such diagnosis may be based on accurate profiling of biomarkers detectable with non-invasive methods for diagnosis of the biological conditions. The present disclosure provides improved systems and methods for accurate diagnosis of biological conditions based on analysis of dynamic biological response data from non-invasively obtained biological samples from subjects. Such improved systems and methods for accurate diagnosis of biological conditions may be based on a combination of dynamic immunohistochemistry profiling of biological samples and artificial intelligence data analysis of such dynamic profiles toward assessment of disease states. The present disclosure addresses these needs, for example, by providing a biological sample biomarker for diagnosis of biological conditions. The biological sample includes a human biological specimen that is associated with incremental growth. Such a biological sample could be a hair shaft, a tooth, and a nail. The non-invasive biomarker of the present disclosure can be used for the diagnosis of young children, even infants younger than one year old. For the disclosed method, the child is less than about 12, 11, 10, 9, 8, 7, 5, 4, 3, 2, or 1 year(s) old. Systems are disclosed and are useful for practising the invention, but are not claimed as such.

The present disclosure provides a method for predicting a subject's diagnostic status with respect to a disease or disorder, comprising: (a) staining a tooth sample of the subject to produce a stained tooth sample; (b) analyzing a fluorescence intensity spatially across the stained tooth sample; and (c) predicting a subject's diagnostic status with respect to a disease or disorder based at least in part on the analysis of the fluorescence intensity. The method of the claimed invention in its general sense is defined by claim 1.

The analyzing comprises obtaining a fluorescence image of the stained tooth sample, and analyzing the fluorescence intensity of the fluorescence image. In some embodiments, obtaining the fluorescence image of the stained tooth sample comprises using an inverted or non-inverted confocal microscope. Staining the tooth sample comprises using a C-reactive protein immunohistochemistry stain. In some embodiments, the method further comprises sectioning the tooth sample. In some embodiments, staining the tooth sample comprises decalcifying the tooth sample.

In some embodiments, the systems and methods disclosed herein may use C-reactive protein fluorescence immunohistochemistry staining alone, or in combination with other techniques. Such techniques may include laser ablation-inductively coupled plasma-mass spectrometry (LA-ICP-MS), Raman spectroscopy or any combination thereof. In some embodiments, combining techniques may improve diagnostic accuracy or precision of a given technique alone. In some embodiments, the addition of LA-ICP-MS may provide a plurality of non-invasive metal metabolism biomarkers of a given biological sample that may complement the diagnostic power of C-reactive protein fluorescence immunohistochemistry data. In some embodiments, the metal metabolism biomarkers may comprise Zinc, Tin, Magnesium, Copper, Iodide, lithium, aluminum, phosphorus, sulfur, calcium, chromium, manganese, iron, cobalt, nickel, arsenic, strontium, cadmium, tin, iodine, barium, mercury, lead, bismuth, molybdenum, or any combination thereof. In some embodiments, the addition of Raman spectroscopy may provide a plurality of spectra indicative of physiological changes induced by disease or external stressors to complement the diagnostic power of C-reactive protein fluorescence immunohistochemistry data.

The disease or disorder for which the claimed method predicts a subject's diagnostic status comprises autism spectrum disorder (ASD). Other diseases or disorders disclosed herein comprise attention deficit/hyperactivity disorder (ADHD), amyotrophic lateral sclerosis (ALS), schizophrenia, irritable bowel disease (IBD), pediatric kidney disease, kidney transplant rejection, pediatric cancer or any combination thereof. In some embodiments, the subject is a human For the claimed method, the subject must be less than about 12, 11, 10, 9, 8, 7, 5, 4, 3, 2, or 1 year(s) old.

In some embodiments, the analyzing comprises generating a temporal profile of inflammation based at least in part on the fluorescence intensity, and analyzing the temporal profile of inflammation. In some embodiments, at least a portion of the temporal profile of inflammation corresponds to a prenatal period of the subject.

In some embodiments, predicting a subject's diagnostic status with respect to a disease or disorder comprises processing the fluorescence intensity using a trained model. In some embodiments, this trained model comprises a plurality of parameters, where the term "parameter" refers to any coefficient or, similarly, any value of an internal or external element (e.g., a weight and/or a hyperparameter) in the model (e.g., where the model is a regressor or a classifier) that can affect (e.g., modify, tailor, and/or adjust) one or more inputs, outputs, and/or functions in the model. For example, in some embodiments, a parameter of a model refers to any coefficient, weight, and/or hyperparameter that can be used to control, modify, tailor, and/or adjust the behavior, learning, and/or performance of the model. In some instances, a parameter is used to increase or decrease the influence of an input (e.g., a feature) to a model. As a nonlimiting example, in some embodiments, a parameter is used to increase or decrease the influence of a node (e.g., of a neural network), where the node includes one or more activation functions. Assignment of parameters to specific inputs, outputs, and/or functions of a model is not limited to any one paradigm for a given model but can be used in any suitable model for a desired performance. In some embodiments, a parameter has a fixed value. In some embodiments, a value of a parameter is manually and/or automatically adjustable. In some embodiments, a value of a parameter is modified by a validation and/or training process for a model (e.g., by error minimization and/or back propagation methods). In some embodiments, a model of the present disclosure includes a plurality of parameters. In some embodiments, the plurality of parameters associated with a model (e.g., an untrained, partially trained, or fully trained model) is n parameters, where: $n \geq 2$; $n \geq 5$; $n \geq 10$; $n \geq 25$; $n \geq 40$; $n \geq 50$; $n \geq 75$; $n \geq 100$; $n \geq 125$; $n \geq 150$; $n \geq 200$; $n \geq 225$; $n \geq 250$; $n \geq 350$; $n \geq 500$; $n \geq 600$; $n \geq 750$; $n \geq 1,000$; $n \geq 2,000$; $n \geq 4,000$; $n \geq 5,000$; $n \geq 7,500$; $n \geq 10,000$; $n \geq 20,000$; $n \geq 40,000$; $n \geq 75,000$; $n \geq 100,000$; $n \geq 200,000$; $n \geq 500,000$, $n \geq 1 \times 10^6$, $n \geq 5 \times 10^6$, or $n \geq 1 \times 10^7$. In some embodiments n is between 10,000 and $1 \times 10^7$, between 100,000 and $5 \times 10^6$, or between 500,000 and $1 \times 10^6$.

In some embodiments, the processing the fluorescence intensity using the trained model comprises extracting features from the fluorescence intensity (e.g., by recurrence quantification analysis), and analyzing the features using the trained model. In some embodiments, the trained model is selected from the group consisting of: a neural network algorithm, a support vector machine algorithm, a decision tree algorithm, an unsupervised clustering algorithm, a supervised clustering algorithm, a regression algorithm, a gradient-boosting algorithm (e.g., a gradient-boosting implementation of a machine learning algorithm such as gradient-boosted decision trees) and any combination thereof. In some embodiments, the trained model comprises a gradient-boosted ensemble model. In some embodiments, the trained model is configured to process one or more features selected from the group consisting of recurrence rates, determinism, mean diagonal length, maximum diagonal length, divergence, Shannon entropy in diagonal length, trend in recurrences, laminarity, trapping time, maximum vertical line length, Shannon entropy in vertical line lengths, mean recurrence time, Shannon entropy in recurrence times, number of the most probable recurrences, mean diagonal length (MDL), recurrence time (RT), Vmax, determinism, Lmax, and any combination thereof. In some embodiments, the one or more features are extracted by applying recurrence quantification analysis (RQA) to fluorescence intensity traces derived from analysis of the sample. In some embodiments, the trained model is configured to process two or more features selected from the group consisting of recurrence rates, determinism, mean diagonal length, maximum diagonal length, divergence, Shannon entropy in diagonal length, trend in recurrences, laminarity, trapping time, maximum vertical line length, Shannon entropy in vertical line lengths, mean recurrence time, Shannon entropy in recurrence times, number of the most probable recurrences, mean diagonal length (MDL), recurrence time (RT), Vmax, determinism, Lmax, and any combination thereof.

In some embodiments, the method further comprises predicting a subject's diagnostic status with respect to a disease or disorder with a sensitivity of at least about 80%. In some embodiments, the method further comprises predicting a subject's diagnostic status with respect to a disease or disorder with a specificity of at least about 80%. In some embodiments, the method further comprises predicting a subject's diagnostic status with respect to a disease or disorder with a positive predictive value of at least about 80%. In some embodiments, the method further comprises predicting a subject's diagnostic status with respect to a disease or disorder with a negative predictive value of at least about 80%. In some embodiments, the method further comprises predicting a subject's diagnostic status with respect to a disease or disorder with an Area Under the Receiver Operating Characteristic (AUROC) of at least about 0.80.

Disclosed but not claimed is a device comprising one or more processors, and memory storing one or more programs for execution by the one or more processors, the one or more programs comprising instructions for:

(a) sampling each respective position in a plurality of positions along a reference line on a biological sample of the subject associated with c-reactive protein of the subject, thereby obtaining a plurality of fluorescence intensity measurements, each fluorescence intensity measurement in the plurality of fluorescence intensity measurements corresponding to a different position in the plurality of positions, and each position in the plurality of positions representing a different period of growth of the biological sample of the subject associated with c-reactive protein;
(b) analyzing each fluorescence intensity across reference line on the biological sample thereby obtaining a first dataset;
(c) deriving a respective second dataset from the corresponding plurality of fluorescence intensity measurements, each respective feature in the corresponding set of features being determined by sequential variability in c-reactive protein fluorescence intensity; and
(d) processing the features using a trained model to determine a likelihood that the subject has the disease or disorder associated with c-reactive protein. In some embodiments, the respective second dataset is derived by applying recurrence quantification analysis or related methods to the corresponding plurality of fluorescence intensity measurements.

Disclosed but not claimed is also a non-transitory computer readable storage medium and one or more computer programs embedded therein, the one or more computer programs comprising instructions which, when executed by a computer system, cause the computer system to perform a method comprising: (a) sampling each respective position in a plurality of positions along a reference line on a biological sample of the subject associated with c-reactive protein of the subject, thereby obtaining a plurality of fluorescence intensity measurements, each fluorescence intensity measurement in the plurality of fluorescence intensity measurements corresponding to a different position in the plurality of positions, and each position in the plurality of positions representing a different period of growth of the biological sample of the subject associated with c-reactive protein; (b) analyzing each fluorescence intensity across reference line on the biological sample thereby obtaining a first dataset; (c) deriving a respective second dataset from the corresponding plurality of fluorescence intensity measurements, each respective feature in the corresponding set of features being determined by sequential variability in c-reactive protein fluorescence intensity; and (d) processing the features using a trained model to determine a likelihood that the subject has the disease or disorder associated with c-reactive protein. In some embodiments, the respective second dataset is derived by applying recurrence quantification analysis or related methods to the corresponding plurality of fluorescence intensity measurements.

Disclosed but not claimed is as well a method for training a model, comprising: at a computer system having one or more processors, and memory storing one or more programs for execution by the one or more processors: (a) for each respective training subject in a plurality of training subjects, wherein a first subset of training subjects in the plurality of training subjects have a first diagnostic status corresponding to having a first biological condition associated with c-reactive protein and a second subset of training subjects in the plurality of training subjects have a second diagnostic status corresponding to not having the first biological condition associated with c-reactive protein: (i) sampling each respective position in a plurality of positions along a reference line on a biological sample of the subject associated with c-reactive protein of the subject, thereby obtaining a plurality of fluorescence intensity measurements, each fluorescence intensity measurement in the plurality of fluorescence intensity measurements corresponding to a different position in the plurality of positions, and each position in the plurality of positions represent a different period of growth of the biological sample of the subject associated with c-reactive protein; (ii) analyzing each fluorescence intensity across reference line on biological sample thereby obtaining a first dataset; and (iii) deriving a respective second dataset from the corresponding plurality of fluorescence intensity measurements, each respective feature in the corresponding set of features being determined by a variation in c-reactive protein fluorescence intensity; and (b) training an untrained or partially untrained model with (i) the corresponding set of features of each respective second dataset of each training subject in the plurality of training subjects and (ii) the corresponding diagnostic status of each training subject in the plurality of training subjects, selected from among the first diagnostic status and the second diagnostic status, thereby obtaining a trained model that provides an indication as to whether a test subject has the first biological condition associated with c-reactive protein based on values for features in a set of features acquired from a biological sample associated with c-reactive protein of the test subject. In some embodiments, the respective second dataset is derived by applying recurrence quantification analysis or related methods to the corresponding plurality of fluorescence intensity measurements.

In some embodiments, the trained model is a neural network algorithm, a support vector machine algorithm, a decision tree algorithm, an unsupervised clustering model algorithm, a supervised clustering model algorithm, a regression model, a gradient-boosting algorithm (e.g., a gradient-boosting implementation of a machine learning algorithm such as gradient-boosted decision trees), or any combination thereof. In some embodiments, the trained machine learning model comprises a gradient-boosted ensemble model. In some embodiments, the trained model predicts outcomes relative to a multinomial distribution. In some embodiments, the trained model predicts outcomes relative to a binomial distribution. In some embodiments, the first biological condition associated with c-reactive protein is selected from the group consisting of autism spectrum disorder (ASD), attention-deficit/hyperactivity disorder (ADHD), amyotrophic lateral sclerosis (ALS), schizophrenia, irritable bowel disease (IBD), pediatric kidney disease, kidney transplant rejection, and pediatric cancer.

In some embodiments, evaluating the test subject for the first biological condition associated with c-reactive protein further includes discriminating between a presence of the first biological condition associated with c-reactive protein and an absence of the first biological condition associated with c-reactive protein. In some embodiments, evaluating the test subject for the first biological condition associated with c-reactive protein further includes discriminating between the first biological condition associated with c-reactive protein and a second biological condition associated with c-reactive protein distinct from the first biological condition associated with c-reactive protein. In some embodiments, the first biological condition is autism spectrum disorder and the second biological condition is neurotypical development; that is, the absence of a neurodevelopmental disorder. In some embodiments, the first biological condition is autism spectrum disorder and the second biological condition is attention-deficit/hyperactivity disorder. In some embodiments, the test subject is a human. In some embodiments, the human may be less than about 12, 11, 10, 9, 8, 7, 5, 4, 3, 2, or 1 year(s) old. The corresponding biological sample associated with c-reactive protein of the respective training subject may be selected from the group consisting of a hair shaft, a tooth, and a nail. A non-claimed corresponding biological sample associated with c-reactive protein of the respective training subject may be the hair shaft and the reference line corresponds to a longitudinal direction of the hair shaft. According to the invention, the corresponding biological sample associated with c-reactive protein of the respective training subject is the tooth and the reference line corresponds to a direction across the growth bands, including the neonatal line of the tooth. In some embodiments, the corresponding plurality of positions is sequenced such that a first position in the corresponding plurality of positions along the corresponding biological sample associated with c-reactive protein of the respective training subject corresponds to a position closest to a tip of the corresponding biological sample associated with c-reactive protein of the respective training subject. In some embodiments, each trace in the corresponding plurality of fluorescence intensity measurements includes a plurality of data points, each data point being an instance of the respective position in the plurality of positions. In some embodiments, the corresponding set of features is selected from the group consisting of recurrence rates, determinism, mean diagonal length, maximum diagonal length, divergence, Shannon entropy in diagonal length, trend in recurrences, laminarity, trapping time, maximum vertical line length, Shannon entropy in vertical line lengths, mean recurrence time, Shannon entropy in recurrence times, number of the most probable recurrences, mean diagonal length (MDL), recurrence time (RT), Vmax, determinism, Lmax, and any combination thereof. In some embodiments, the features are derived from recurrence quantification analysis or related computational analysis of the fluorescence trace . In some embodiments, the corresponding plurality of positions includes at least 1000, 1500, 2000, 2500, 3000, 3500, 4000, 4500, 5000, 6000, 7000, 8000, 9000, 10000, 12000, 14000, 16000, 18000, 20000, or more than 20000 positions.

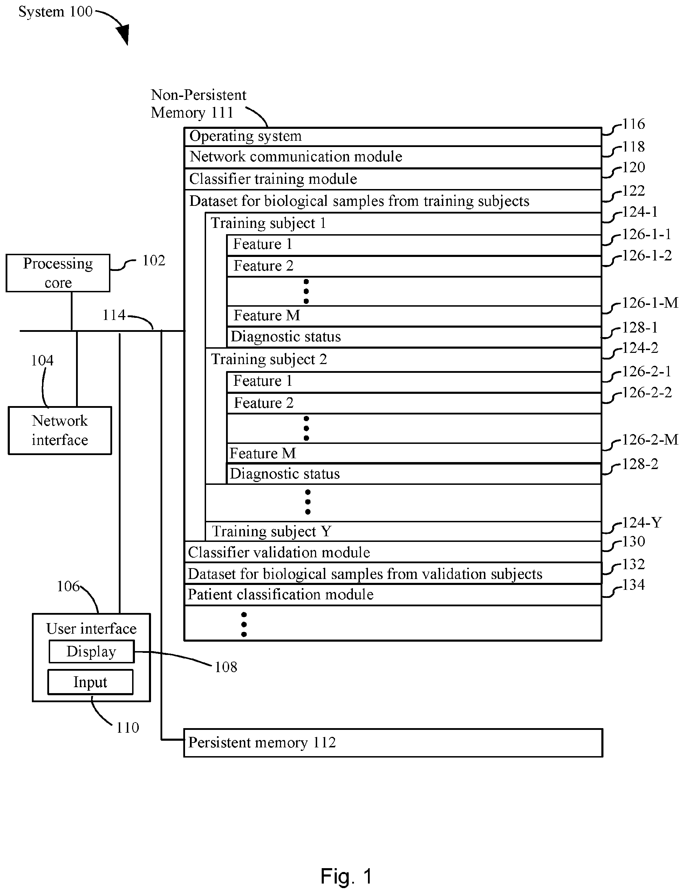

Details of an exemplary system are described in conjunction with FIG. 1, which shows an example of a block diagram of a computing device 100. The device 100 in some implementations includes one or more processing units CPU(s) 102 (also referred to as processors), one or more network interfaces 104, a user interface 106, a non-persistent memory 111, a persistent memory 112, and one or more communication buses 114 for interconnecting these components. The one or more communication buses 114 optionally include circuitry (sometimes called a chipset) that interconnects and controls communications between system components. The non-persistent memory 111 typically includes high-speed random access memory, such as DRAM, SRAM, DDR RAM, ROM, EEPROM, flash memory, whereas the persistent memory 112 typically includes CD-ROM, digital versatile disks (DVD) or other optical storage, magnetic cassettes, magnetic tape, magnetic disk storage or other magnetic storage devices, magnetic disk storage devices, optical disk storage devices, flash memory devices, or other non-volatile solid state storage devices. The persistent memory 112 optionally includes one or more storage devices remotely located from the CPU(s) 102. The persistent memory 112, and the non-volatile memory device(s) within the non-persistent memory 112, comprise non-transitory computer readable storage medium.

In some implementations, the non-persistent memory 111 or alternatively the non-transitory computer readable storage medium stores the following programs, modules and data structures, or a subset thereof, sometimes in conjunction with the persistent memory 112: an optional operating system 116, which includes procedures for handling various basic system services and for performing hardware dependent tasks; an optional network communication module (or instructions) 118 for connecting the system 100 with other devices and/or a communication network 104; an optional classifier training module 120 for training models for evaluating a subject for a biological condition; an optional data store 122 for datasets for biological samples from training subjects, including feature data for one or more training subjects 124, where the feature data includes a parameter associated with each of features 126, and diagnostic status 128 (e.g., an indication that a respective training subject has been diagnosed with a biological condition or has not been diagnosed with a biological condition); an optional classifier validation module 130 for validating models that distinguish the a biological condition; an optional data store 132 for datasets for biological samples from validation subjects; and an optional patient classification module 134 for classifying a subject as having a biological condition, e.g., as trained using classifier training module 120.

In various implementations, one or more of the above identified elements are stored in one or more of the previously mentioned memory devices, and correspond to a set of instructions for performing a function described above. The above identified modules, data, or programs (e.g., sets of instructions) need not be implemented as separate software programs, procedures, datasets, or modules, and thus various subsets of these modules and data may be combined or otherwise re-arranged in various implementations. In some implementations, the non-persistent memory 111 optionally stores a subset of the modules and data structures identified above. Furthermore, optionally, the memory stores additional modules and data structures not described above. Alternatively, one or more of the above identified elements is stored in a computer system, other than that of visualization system 100, that is addressable by visualization system 100 so that visualization system 100 may retrieve all or a portion of such data when needed.

The system 100 may be connected to, or includes, one or more analytical devices for performing chemical analyzes. For example, the optional network communication module (or instructions) 118 is configured to connect the system 100 with the one or more analytical devices, e.g., via the communication network 104. Optionally, the one or more analytical devices include a laser ablation-inductively coupled plasma-mass spectrometer (LA-ICP-MS), a fluorescence image sensor, or a Raman spectrometer.

Although **FIG. 1** depicts a "system 100," the figure is intended more as functional description of the various features which may be present in computer systems than as a structural schematic of the implementations described herein. In practice, and as recognized by those of ordinary skill in the art, items shown separately may be combined and some items may be separated. Moreover, although FIG. 1 depicts certain data and modules in non-persistent memory 111, some or all of these data and modules may be in persistent memory 112.

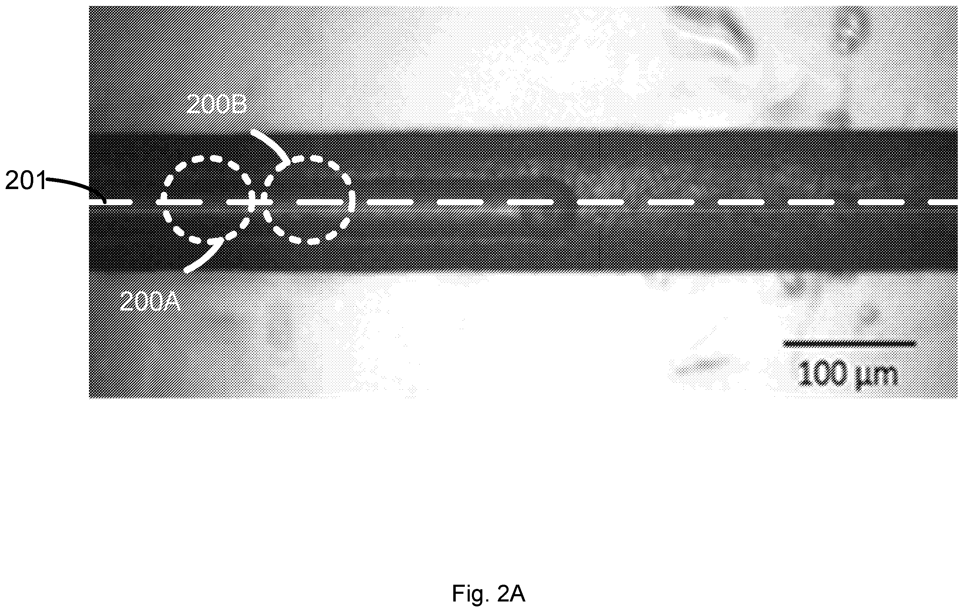

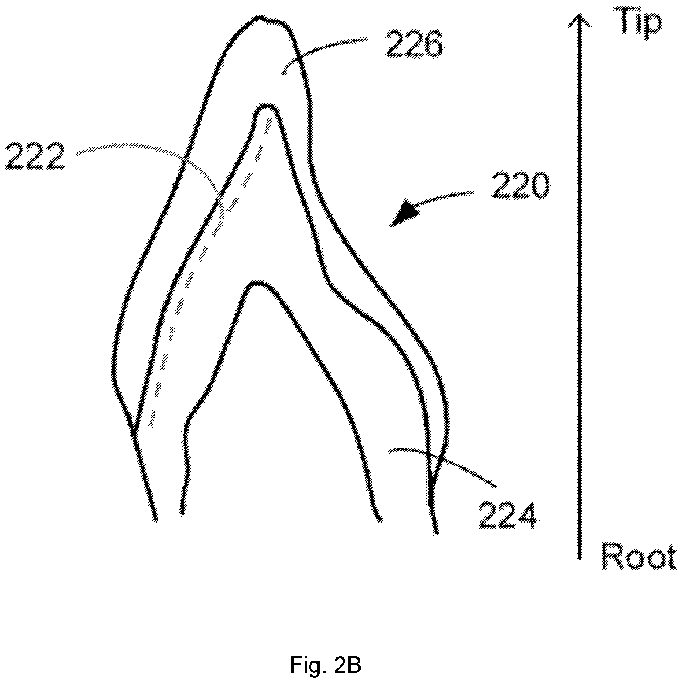

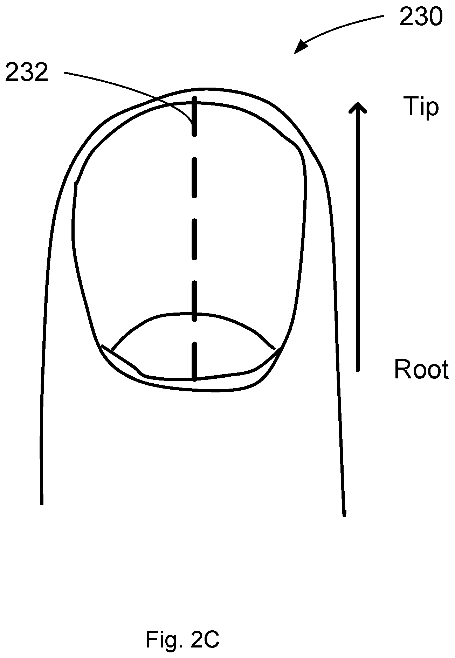

A disclosed but not claimed further method step is obtaining a biological sample (e.g., a strand of hair including a hair shaft). The subject may be a human. Preferably, the subject is a child aged equal to or below 5 years (e.g., the child is aged equal to or below 5 years, 4 years, 3 years, 2 years, 1 year, 9 months, 6 months, 3 months, or 1 month). Alternatively, the subject is an adult. FIG. 2A shows an example of a hair sample of a subject including a hair shaft. The hair sample may be simply cut from the subject (e.g., with help of scissors). The method of obtaining the hair sample may be non-invasive. The obtained hair sample may have a minimum length of 1 cm (e.g., the hair sample is 1 cm, 2 cm, 3 cm, 4 cm, or 5 cm long). The hair sample may include any portion of a hair (e.g., a tip or a portion between the tip and a follicle). In particular, there is no special requirement for the hair sample to include the hair follicle. FIG. 2B shows an example of a tooth sample of a subject. FIG. 2C shows an example of a nail sample of a subject. In instances of a nail or a hair, obtaining a biological sample may refer to positioning the subject such that the nail or the hair may be sampled. The nail sample may comprise a whole nail or a nail clipping.

The obtained biological sample may be pre-processed, such as being pre-treated by washing the biological sample with one or more solvents and/or surfactants and drying. In an instance that the biological sample is a hair, the hair sample may be washed in a solution of TRITON X-100® and ultrapure metal free water (e.g., MILLI-Q® water) and dried overnight in an oven (e.g., at 60 degrees Celsius). The pre-treatment may further include preparing the hair shaft for a measurement by placing the hair shaft on a glass slide (e.g., a microscopic glass slide) with an adhesive film (e.g., a double-sided tape). The hair shaft may be positioned such that the hair shaft is substantially straight. The glass slide with the hair shaft may be placed into or in the vicinity of a measurement system (e.g., a laser ablation-inductively coupled plasma-mass spectrometer (LA-ICP-MS), a fluorescence image sensor, or a Raman spectrometer) for performing analysis. In an instance that the biological sample is a tooth or a nail, a surface of the biological sample may be cleaned (e.g., by surfactant, water, or one or more solvents). In some cases, the sample may be decalcified prior to, after, immediately before, or any combination of time frames with respect to performing analysis, described elsewhere herein. In some cases, decalcifying the sample may comprise the steps of: (a) soaking a tooth in a solution of ethylenediaminetetraacetic acid (EDTA), where the EDTA may comprise a pH of about 7.0 to about 7.4 for a period of up to about 5 weeks; (b) weighing the tooth with a weekly frequency; (c) removing the sample when the change in weight of the tooth plateaus. The sample may be placed into or in the vicinity of a measurement system (e.g., a laser ablation-inductively coupled plasma-mass spectrometer (LA-ICP-MS), a fluorescence image sensor, or a Raman spectrometer) for performing analysis.

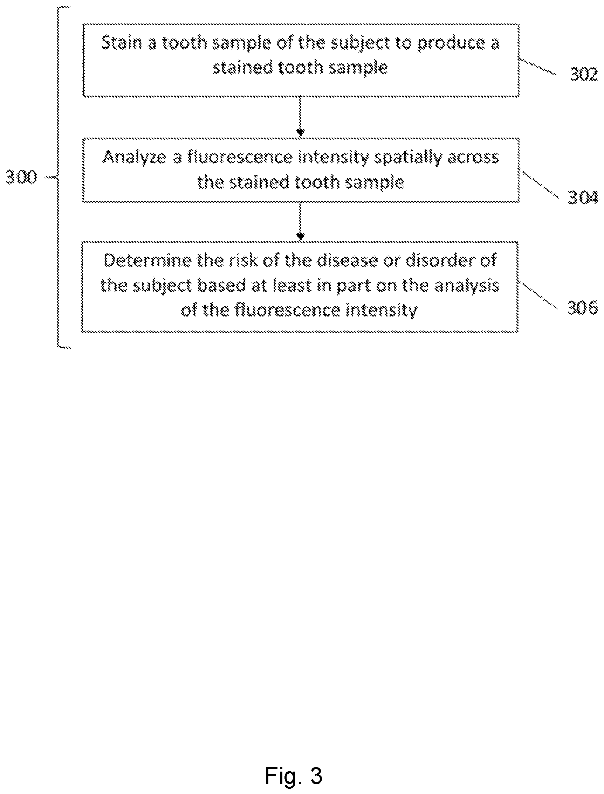

FIG. 3 shows a flow chart of a method 300 for evaluating a subject for a biological condition, such as a method for predicting a subject's diagnostic status with respect to of a disease or disorder. The method 300 may comprise staining a tooth sample of the subject to produce a stained tooth sample (as in operation 302). Next, the method 300 may comprise analyzing a fluorescence intensity spatially across the stained tooth sample (as in operation 304). Next, the method 300 may comprise predicting a subject's diagnostic status with respect to a disease or disorder based at least in part on the analysis of the fluorescence intensity (as in operation 306).

According to the invention, the analyzing comprises obtaining a fluorescence image of the stained tooth sample, and analyzing the fluorescence intensity of the fluorescence image. In some embodiments, obtaining the fluorescence image of the stained tooth sample comprises using an inverted or non-inverted confocal microscope. Staining the tooth sample comprises using a C-reactive protein immunohistochemistry stain. In some embodiments, the method further comprises sectioning the tooth sample. In some embodiments, staining the tooth sample comprises decalcifying the tooth sample.

In some embodiments, the analyzing comprises generating a temporal profile of inflammation based at least in part on the fluorescence intensity, and analyzing the temporal profile of inflammation. In some embodiments, at least a portion of the temporal profile of inflammation corresponds to a prenatal period of the subject.

Measurement data may be collected from the biological sample sequentially at a plurality of positions along the biological sample. In some embodiments, the plurality of positions along the reference line of the biological sample includes at least 100 positions (e.g., 1000, 1500, 2000, 2500, 3000, 3500, 4000, 4500, 5000, 6000, 7000, 8000, 9000, 10000, 12000, 14000, 16000, 18000, 20000, or more than 20000 positions). In some embodiments, the respective positions are adjacent to each other. By this method, each area corresponding to a distinct position on the biological sample may be thereby associated with a dynamic (e.g., time-varying) abundance measurement. In some embodiments, the respective positions are separated by a predefined distance. In some embodiments, the sampling is performed along the reference line of the biological sample starting from a respective position nearest to the tip of the biological sample such as hair sample (e.g., at a position that corresponds to the youngest age of the subject). In general, the sampling can be performed starting from a respective position nearest to the tip or the root, as long as the direction of the sampling is known, and an appropriate trained model is used for the analyses.

The sampling may produce sets of data points. Each set of data points may correspond to a measurement (e.g., an abundance or concentration) of a substance that is indicative of a dynamic biological response measured at a plurality of positions along the biological sample. Each position on the reference line of the biological sample may correspond to a specific time of growth of the biological sample.

In some embodiments, in an instance of a biological sample of a tooth, the reference line may comprise 240 - 510 days of growth (e.g., the period of tooth crown formation depending on tooth type). In some embodiments, each position along the reference line may correspond to about 1 to about 0.5 micrometers. Alternatively, or in addition to, the biological sample may comprise a hair shaft, where the position along the reference line corresponds to approximately 5 min of growth (e.g., the period of hair growth calculated using a 1 micrometer resolution and an average rate of hair growth 1 cm per month). By correlating the plurality of positions along the reference line of the biological sample to corresponding time periods of the growth, a first dataset including a plurality of traces is obtained. Each trace includes a time-dependent abundance of a measurement (e.g., an abundance or concentration) of a substance that is indicative of a dynamic biological response measured from the biological sample. For example, the distance between positions may correspond to an estimated growth of the biological sample (e.g., biological time). For example, abundance may be measured for a tooth sample along up to about 8 millimeters (mm) distance, which corresponds to a biological time of approximately 240 - 510 days. Alternatively, or in addition to, abundance may be measured for a hair sample along a 1.2cm distance, which corresponds to a biological time of approximately 35 days. The biological time may be estimated by using an average rate of hair growth (e.g., 1 cm per month).

In some embodiments, data analysis may be performed on the traces corresponding to a time-dependent abundance (e.g., a time-dependent concentration) of a substance that is indicative of a dynamic biological response measured from the biological sample. This may comprise customized operations to clean the data (e.g., smoothening the data over a time span, and/or removing data points that are higher or lower than a predetermined threshold). In some embodiments, the data analysis includes removing, from the traces, data points that have a mean absolute difference between adjacent data points that is at least one, two, or three times a standard deviation of the mean absolute difference between adjacent points.

In some embodiments, the data analysis further includes normalizing each trace against an internal standard. For example, a measured substance detected in the samples that is evenly incorporated during the development/growth of a biological sample that does not fluctuate with environmental exposures (e.g., diet) can serve as an internal standard.

In some embodiments, the data analysis further includes performing recurrence quantification analysis (RQA) on the time-dependent traces to obtain a set of features that describe dynamical periodical characteristics of the traces. RQA measures variability in the time-dependent traces. RQA involves the estimation of features that describe periodic properties in a given waveform, which include determinism, entropy, mean diagonal length (MDL), laminarity, entropy, trapping time (TT), recurrence time (RT), Vmax, and Lmax, each of which captures varying aspects of signal dynamics, as described in accompanying references. Methods and features of RQA are described, for example, by Webber et al. in "Simpler Methods Do It Better: Success of Recurrence Quantification Analysis as a General Purpose Data Analysis Tool," Physics Letters A 373, 3753-3756 (2009) and by Marwan et al. in "Recurrence Plots for the Analysis of Complex Systems," Physics Reports 438, 237-239 (2007). In some embodiments, the time-dependent traces are analyzed by using other analytical methods, such as Fourier Transformations, Wavelet Analysis, and Cosinor analysis. Such techniques can be applied to derive similar metrics, including spectral analysis of frequency components and their associated power. These metrics and associated derivative measures may be used in place of the features derived from RQA to analyze the time-dependent traces obtained from biological samples for purposes of predictive classification.

The RQA includes construction of recurrence plots that visualize and analyze dynamical temporal structures in respective obtained traces. Such recurrence plots may illustrate phasic processes in sequential measurements by plotting a given sequence against a time-lagged derivation of that sequence. From the one dimensional trace measured from the hair shaft, additional dimensions are computationally derived to embed the trace in a higher dimensional space referred to as a phase portrait, where t refers to the values of the original trace, and dimensions (t+τ) and (t+2τ) are derived from lagging the original time series by interval τ. Subsequent analyses are then undertaken on the embedded phase portrait to construct recurrence plots and to undertake recurrence quantification analysis. A recurrence plot may be derived from the phase portrait through the application of a threshold function to each point in the phase portrait; on the corresponding recurrence plot, consisting of a square binary matrix, typically represented as white or black space, a given point is assigned a value of 1 at each temporal interval wherein another point in the phase-portrait shares the spatial limits of the assigned threshold boundary. The RQA method is applied to the recurrence plot to examine the interval of delay between states in a given system, with a black point reflecting the temporal interval when a system revisits the same state. Periodic processes, where a system successively reiterates a given pattern of states, will manifest in a recurrence plot as diagonal black lines, whereas periods of stability will manifest as square structures, spurious repetitions as black dots, and, unique events as white space.

In some embodiments, the recurrence plots are constructed for traces of a single substance or a combination of two substances (e.g., in order to visualize an interactive periodic pattern of two substances; this can be referred to as cross-recurrence quantification analysis, or joint-recurrence quantification analysis). In some embodiments, the recurrence plots are constructed for a combination of three or more substances.

In some embodiments, the data analysis includes analyzing the recurrence plots to obtain a set of features associated with the recurrence plots. The features, which interchangeably can be termed "rhythmicity features," or "dynamic features," provide a quantitative measure describing the periodicity, predictability, and transitivity present in the plurality of traces. The features are selected from a set including recurrence rates, determinism, mean diagonal length, maximum diagonal length, divergence, Shannon entropy in diagonal length, trend in recurrences, laminarity, trapping time, maximum vertical line length, Shannon entropy in vertical line lengths, mean recurrence time, Shannon entropy in recurrence times, number of the most probable recurrences, mean diagonal length (MDL), laminarity, entropy, trapping time (TT), recurrence time (RT), Vmax, Lmax, and any combination thereof.

In some embodiments, the data analysis further includes inputting the obtained set of features to a trained model. In some embodiments, the trained model includes a predictive computational algorithm to obtain a probability for the subject having a biological condition. In some embodiments, the predictive computational algorithm performs the following calculation:

$$p(subject) = \frac{1}{1 + e^{-(\alpha + \beta_1 x_1 + \cdots + \beta_k x_k)}}$$

where *p(subject)* is the probability that the subject has the first biological condition, e is Euler's number, a is a calculated parameter associated with the probability that the subject has the biological condition when $\beta_1 x_1 + ... + \beta_k x_k$ equals to zero, $x_1, ..., x_k$ corresponds to a value derived for each feature in the set of features, the set of features including features from 1 through k, and $\beta_1, ..., \beta_k$ corresponds to a weight parameter associated with each feature in the set of features including features from 1 through k.

The weight parameters $\beta_1, ..., \beta_x$ may be defined based on model training. The probability *p(subject)* may be provided as a number ranging from 0 to 1, where 1 corresponds to a 100% probability that the subject has a biological condition.

The data analysis may include applying a threshold to the obtained probability p(subject). If the obtained probability p(subject) is above the threshold, the subject is evaluated as having the biological condition. If the obtained probability is below the threshold, the subject is evaluated as not having the biological condition. The threshold may be between about 0.3 and 0.6 (e.g., the predetermined threshold is about 0.3, 0.35, 0.4, 0.45, 0.5, 0.55, or 0.6). The value assigned for a probabilistic threshold may be predetermined, or estimated during the training of the model through the use of receiver-operating-characteristic (ROC) charts, with the optimal threshold used corresponding to the value which yields the maximum area-under-the-curve (ROC-AUC). The obtained probability may be expressed in terms of associated odds (e.g., odds ratio (OR), which may be derived from a probability such that OR=p/(1-p)). For example, the evaluation includes evaluating odds that the subject has the biological condition.

The data analysis may include discriminating a first biological condition from an alternative condition, e.g., a second, biological condition. The alternative condition may be associated with no known condition (e.g., a neurotypical condition (NT)). The first biological condition may be associated with autism spectrum disorder (ASD) and the alternative condition is associated with an attention-deficit/hyperactivity disorder (ADHD). Alternatively, the alternative condition may be any other neurodevelopmental condition, or a comorbid diagnosis for two neurodevelopmental conditions. Therefore, the data analysis may be capable of discriminating between two neurodevelopmental conditions (e.g., between autism spectrum disorder and ADHD, or between ASD and co-morbid (CM) cases diagnosed for both ASD and ADHD).

Health care providers, such as physicians and treating teams of a patient may have access to patient data (e.g., dynamic biological response data or other health data), and/or predictions or assessments generated from such data. Based on the data analysis results, health care providers may determine clinical decisions or outcomes.

For example, a physician may instruct that patient undergo one or more clinical tests at the hospital or other clinical site, based at least in part on a predicted disease or disorder in the subject. These instructions may be provided when a certain pre-determined criterion is met (e.g., a minimum threshold for a likelihood of the disease or disorder).

Such a minimum threshold may be, for example, at least about a 5% likelihood, at least about a 10% likelihood, at least about a 20% likelihood, at least about a 25% likelihood, at least about a 30% likelihood, at least about a 35% likelihood, at least about a 40% likelihood, at least about a 45% likelihood, at least about a 50% likelihood, at least about a 55% likelihood, at least about a 60% likelihood, at least about a 65% likelihood, at least about a 70% likelihood, at least about a 75% likelihood, at least about an 80% likelihood, at least about a 85% likelihood, at least about a 90% likelihood, at least about a 95% likelihood, at least about a 96% likelihood, at least about a 97% likelihood, at least about a 98% likelihood, or at least about a 99% likelihood.

As another example, a physician may prescribe a therapeutically effective dose of a treatment (e.g., drug), a clinical procedure, or further clinical testing to be administered to the patient based at least in part on a predicted disease or disorder in the subject. For example, the physician may prescribe an anti-inflammatory therapeutic in response to an indication of inflammation in the patient.

## Models

The methods and systems of the present disclosure may utilize or access external capabilities of artificial intelligence techniques to develop signatures for various diseases or disorders. These signatures may be used to accurately predict diseases or disorders (e.g., months or years earlier than with standard of clinical care). Using such a predictive capability, health care providers (e.g., physicians) may be able to make informed, accurate risk-based decisions, thereby improving quality of care and monitoring provided to patients.

The methods and systems of the present disclosure may analyze acquired dynamic biological response data from a subject (patient) to generate a likelihood of the subject having a disease or disorder. For example, the system may apply a trained (e.g., prediction) algorithm to the acquired dynamic biological response data to generate the likelihood of the subject having a disease or disorder. The trained algorithm may comprise an artificial intelligence-based model, such as a classifier or regressor, configured to process the acquired dynamic biological response data to generate the likelihood of the subject having the disease or disorder. The model may be trained using clinical datasets from one or more cohorts of patients, e.g., using clinical health data and/or dynamic biological response data of the patients as inputs and known clinical health outcomes (e.g., disease or disorder) of the patients as outputs to the model.

The model may comprise one or more machine learning algorithms. Examples of machine learning algorithms may include a support vector machine (SVM), a naïve Bayes classification, a random forest, a neural network (such as a deep neural network (DNN), a recurrent neural network (RNN), a deep RNN, a long short-term memory (LSTM) recurrent neural network (RNN), or a gated recurrent unit (GRU), or other supervised learning algorithm or unsupervised machine learning, statistical, or deep-learning algorithm for classification and regression. The model may likewise involve the estimation of ensemble models, comprised of multiple predictive models, and utilize techniques such as gradient boosting, for example in the construction of gradient-boosting decision trees. The model may be trained using one or more training datasets corresponding to patient data.

Training datasets may be generated from, for example, one or more cohorts of patients having common clinical characteristics (features) and clinical outcomes (labels). Training datasets may comprise a set of features and labels corresponding to the features. Features may correspond to algorithm inputs comprising dynamic biological response data, patient demographic information derived from electronic medical records (EMR), and medical observations. Features may comprise clinical characteristics such as, for example, certain ranges or categories of dynamic biological response data. Features may comprise patient information such as patient age, patient medical history, other medical conditions, current or past medications, and time since the last observation. For example, a set of features collected from a given patient at a given time point may collectively serve as a signature, which may be indicative of a health state or status of the patient at the given time point.

For example, ranges of dynamic biological response data and other health measurements may be expressed as a plurality of disjoint continuous ranges of continuous measurement values, and categories of dynamic biological response data and other health measurements may be expressed as a plurality of disjoint sets of measurement values (e.g., {"high", "low"}, {"high", "normal"}, {"low", "normal"}, {"high", "borderline high", "normal", "low"}, etc.). Clinical characteristics may also include clinical labels indicating the patient's health history, such as a diagnosis of a disease or disorder, a previous administration of a clinical treatment (e.g., a drug, a surgical treatment, chemotherapy, radiotherapy, immunotherapy, etc.), behavioral factors, or other health status (e.g., hypertension or high blood pressure, hyperglycemia or high blood glucose, hypercholesterolemia or high blood cholesterol, history of allergic reaction or other adverse reaction, etc.).

Labels may comprise clinical outcomes such as, for example, a presence, absence, diagnosis, or prognosis of a disease or disorder in the subject (e.g., patient). Clinical outcomes may include a temporal characteristic associated with the presence, absence, diagnosis, or prognosis of the disease or disorder in the patient. For example, temporal characteristics may be indicative of the patient having had an occurrence of the disease or disorder within a certain period of time after a previous clinical outcome (e.g., being discharged from the hospital, being administered a treatment such as medication, undergoing a clinical procedure such as surgical operation, etc.). Such a period of time may be, for example, about 1 hour, about 2 hours, about 3 hours, about 4 hours, about 6 hours, about 8 hours, about 10 hours, about 12 hours, about 14 hours, about 16 hours, about 18 hours, about 20 hours, about 22 hours, about 24 hours, about 2 days, about 3 days, about 4 days, about 5 days, about 6 days, about 7 days, about 10 days, about 2 weeks, about 3 weeks, about 4 weeks, about 1 month, about 2 months, about 3 months, about 4 months, about 6 months, about 8 months, about 10 months, about 1 year, or more than about 1 year.

Input features may be structured by aggregating the data into bins or alternatively using a one-hot encoding. Inputs may also include feature values or vectors derived from the previously mentioned inputs, such as cross-correlations calculated between separate dynamic biological response data or other measurements over a fixed period of time, and the discrete derivative or the finite difference between successive measurements. Such a period of time may be, for example, about 1 hour, about 2 hours, about 3 hours, about 4 hours, about 6 hours, about 8 hours, about 10 hours, about 12 hours, about 14 hours, about 16 hours, about 18 hours, about 20 hours, about 22 hours, about 24 hours, about 2 days, about 3 days, about 4 days, about 5 days, about 6 days, about 7 days, about 10 days, about 2 weeks, about 3 weeks, about 4 weeks, about 1 month, about 2 months, about 3 months, about 4 months, about 6 months, about 8 months, about 10 months, about 1 year, or more than about 1 year.

Training records may be constructed from sequences of observations. Such sequences may comprise a fixed length for ease of data processing. For example, sequences may be zero-padded or selected as independent subsets of a single patient's records.

The model may process the input features to generate output values comprising one or more classifications, one or more predictions, or a combination thereof. For example, such classifications or predictions may include a binary classification of a healthy/normal health state (e.g., absence of a disease or disorder) or an adverse health state (e.g., presence of a disease or disorder), a classification between a group of categorical labels (e.g., 'no disease or disorder', 'apparent disease or disorder', and 'likely disease or disorder'), a likelihood (e.g., relative likelihood or probability) of developing a particular disease or disorder, a score indicative of a presence of disease or disorder, a score indicative of a level of systemic inflammation experienced by the patient, a 'risk factor' for the likelihood of mortality of the patient, a prediction of the time at which the patient is expected to have developed the disease or disorder, and a confidence interval for any numeric predictions. Various machine learning techniques may be cascaded such that the output of a machine learning technique may also be used as input features to subsequent layers or subsections of the model.

In order to train the model (e.g., by determining weights and correlations of the model) to generate real-time classifications or predictions, the model can be trained using datasets. Such datasets may be sufficiently large to generate statistically significant classifications or predictions. For example, datasets may comprise: databases of de-identified data including dynamic biological response data and other measurements, and dynamic biological response data and other measurements from a hospital or other clinical setting.

Datasets may be split into subsets (e.g., discrete or overlapping), such as a training dataset, a development dataset, and a test dataset. For example, a dataset may be split into a training dataset comprising 80% of the dataset and a test dataset comprising 20% of the dataset. The training dataset may comprise about 10%, about 20%, about 30%, about 40%, about 50%, about 60%, about 70%, about 80%, or about 90% of the dataset. The development dataset may comprise about 10%, about 20%, about 30%, about 40%, about 50%, about 60%, about 70%, about 80%, or about 90% of the dataset. The test dataset may comprise about 10%, about 20%, about 30%, about 40%, about 50%, about 60%, about 70%, about 80%, or about 90% of the dataset. Training sets (e.g., training datasets) may be selected by random sampling of a set of data corresponding to one or more patient cohorts to ensure independence of sampling. Alternatively, training sets (e.g., training datasets) may be selected by proportionate sampling of a set of data corresponding to one or more patient cohorts to ensure independence of sampling.

To improve the accuracy of model predictions and reduce overfitting of the model, the datasets may be augmented to increase the number of samples within the training set. For example, data augmentation may comprise rearranging the order of observations in a training record. To accommodate datasets having missing observations, methods to impute missing data may be used, such as forward-filling, back-filling, linear interpolation, and multi-task Gaussian processes. Datasets may be filtered to remove confounding factors. For example, within a database, a subset of patients may be excluded.

The model may comprise one or more neural networks, such as a neural network, a convolutional neural network (CNN), a deep neural network (DNN), a recurrent neural network (RNN), or a deep RNN. The recurrent neural network may comprise units which can be long short-term memory (LSTM) units or gated recurrent units (GRU). For example, the model may comprise an algorithm architecture comprising a neural network with a set of input features such as vital sign and other measurements, patient medical history, and/or patient demographics. Neural network techniques, such as dropout or regularization, may be used during training the model to prevent overfitting. The neural network may comprise a plurality of sub-networks, each of which is configured to generate a classification or prediction of a different type of output information (e.g., which may be combined to form an overall output of the neural network). The model may alternatively utilize statistical or related algorithms including random forest, classification and regression trees, support vector machines, discriminant analyses, regression techniques, as well as ensemble and gradient-boosted variations thereof.

When the model generates a classification or a prediction of a disease or disorder, a notification (e.g., alert or alarm) may be generated and transmitted to a health care provider, such as a physician, nurse, or other member of the patient's treating team within a hospital. Notifications may be transmitted via an automated phone call, a short message service (SMS) or multimedia message service (MMS) message, an e-mail, or an alert within a dashboard. The notification may comprise output information such as a prediction of a disease or disorder, a likelihood of the predicted disease or disorder, a time until an expected onset of the disease or disorder, a confidence interval of the likelihood or time, or a recommended course of treatment for the disease or disorder.

To validate the performance of the model, different performance metrics may be generated. For example, an area under the receiver-operating curve (AUROC) may be used to determine the diagnostic capability of the model. For example, the model may use classification thresholds which are adjustable, such that specificity and sensitivity are tunable, and the receiver-operating curve (ROC) can be used to identify the different operating points corresponding to different values of specificity and sensitivity.

In some cases, such as when datasets are not sufficiently large, cross-validation may be performed to assess the robustness of a model across different training and testing datasets.

To calculate performance metrics such as sensitivity, specificity, accuracy, positive predictive value (PPV), negative predictive value (NPV), AUPRC, AUROC, or similar, the following definitions may be used. A "false positive" may refer to an outcome in which a positive outcome or result has been incorrectly or prematurely generated (e.g., before the actual onset of, or without any onset of, the disease or disorder). A "true positive" may refer to an outcome in which positive outcome or result has been correctly generated, when the patient has the disease or disorder (e.g., the patient shows symptoms of the disease or disorder, or the patient's record indicates the disease or disorder). A "false negative" may refer to an outcome in which a negative outcome or result has been generated, but the patient has the disease or disorder (e.g., the patient shows symptoms of the disease or disorder, or the patient's record indicates the disease or disorder). A "true negative" may refer to an outcome in which a negative outcome or result has been generated (e.g., before the actual onset of, or without any onset of, the disease or disorder).

The model may be trained until certain predetermined conditions for accuracy or performance are satisfied, such as having minimum desired values corresponding to diagnostic accuracy measures. For example, the diagnostic accuracy measure may correspond to prediction of a likelihood of occurrence of a disease or disorder in the subject. As another example, the diagnostic accuracy measure may correspond to prediction of a likelihood of deterioration or recurrence of a disease or disorder for which the subject has previously been treated. Examples of diagnostic accuracy measures may include sensitivity, specificity, positive predictive value (PPV), negative predictive value (NPV), accuracy, area under the precision-recall curve (AUPRC), and area under the curve (AUC) of a Receiver Operating Characteristic (ROC) curve (AUROC) corresponding to the diagnostic accuracy of detecting or predicting a disease or disorder.

For example, such a pre-determined condition may be that the sensitivity of predicting the disease or disorder comprises a value of, for example, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%.

As another example, such a pre-determined condition may be that the specificity of predicting the disease or disorder comprises a value of, for example, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%.

As another example, such a pre-determined condition may be that the positive predictive value (PPV) of predicting the disease or disorder comprises a value of, for example, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%.

As another example, such a pre-determined condition may be that the negative predictive value (NPV) of predicting the disease or disorder comprises a value of, for example, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%.

As another example, such a pre-determined condition may be that the area under the curve (AUC) of a Receiver Operating Characteristic (ROC) curve (AUROC) of predicting the disease or disorder comprises a value of at least about 0.50, at least about 0.55, at least about 0.60, at least about 0.65, at least about 0.70, at least about 0.75, at least about 0.80, at least about 0.85, at least about 0.90, at least about 0.95, at least about 0.96, at least about 0.97, at least about 0.98, or at least about 0.99.

As another example, such a pre-determined condition may be that the area under the precision-recall curve (AUPRC) of predicting the disease or disorder comprises a value of at least about 0.10, at least about 0.15, at least about 0.20, at least about 0.25, at least about 0.30, at least about 0.35, at least about 0.40, at least about 0.45, at least about 0.50, at least about 0.55, at least about 0.60, at least about 0.65, at least about 0.70, at least about 0.75, at least about 0.80, at least about 0.85, at least about 0.90, at least about 0.95, at least about 0.96, at least about 0.97, at least about 0.98, or at least about 0.99.

In some embodiments, the model may be trained or configured to predict the disease or disorder with a sensitivity of at least about 80%, at least about 85%, at least about 90%, at least about 95%, at least about 96%, at least about 97%, at least about 98%, or at least about 99%.

In some embodiments, the model may be trained or configured to predict the disease or disorder with a specificity of at least about 80%, at least about 85%, at least about 90%, at least about 95%, at least about 96%, at least about 97%, at least about 98%, or at least about 99%.

In some embodiments, the model may be trained or configured to predict the disease or disorder with a positive predictive value (PPV) of at least about 80%, at least about 85%, at least about 90%, at least about 95%, at least about 96%, at least about 97%, at least about 98%, or at least about 99%.

In some embodiments, the model may be trained or configured to predict the disease or disorder with a negative predictive value (NPV) of at least about 80%, at least about 85%, at least about 90%, at least about 95%, at least about 96%, at least about 97%, at least about 98%, or at least about 99%.

In some embodiments, the trained model may be trained or configured to predict the disease or disorder with an area under the curve (AUC) of a Receiver Operating Characteristic (ROC) curve (AUROC) of at least about 0.80, at least about 0.85, at least about 0.90, at least about 0.95, at least about 0.96, at least about 0.97, at least about 0.98, or at least about 0.99.

In some embodiments, the model may be trained or configured to predict the disease or disorder with an area under the precision-recall curve (AUPRC) of at least about 0.10, at least about 0.15, at least about 0.20, at least about 0.25, at least about 0.30, at least about 0.35, at least about 0.40, at least about 0.45, at least about 0.50, at least about 0.55, at least about 0.60, at least about 0.65, at least about 0.70, at least about 0.75, at least about 0.80, at least about 0.85, at least about 0.90, at least about 0.95, at least about 0.96, at least about 0.97, at least about 0.98, or at least about 0.99.

The training data sets may be collected from training subjects (e.g., humans). Each training has a diagnostic status indicating that they have either been diagnosed with the biological condition, or have not been diagnosed with the biological condition. The training subjects may be children aged equal to, or below, 5 years (e.g., equal to or below 5 years, 4 years, 3 years, 2 years, 1 year, 9 months, 6 months, 3 months or 1 month). The following training procedure may be performed for each training subject in a plurality of training subjects.

Training data (e.g., dynamic IHC data) may be generated from biological samples of training subjects. For each biological sample, a plurality of positions of a reference line on a biological sample of the training subject may be sampled in order to generate measurements therefrom, thereby obtaining a plurality of dynamic biological response samples. Each dynamic biological response sample in the corresponding plurality of dynamic biological response samples corresponds to a different position in the corresponding plurality of positions, and each position in the corresponding plurality of positions represents a different period of growth of the corresponding biological sample. Next, each respective position of the biological sample is analyzed (e.g., using a laser ablation-inductively coupled plasma-mass spectrometer (LA-ICP-MS), a fluorescence image sensor, or a Raman spectrometer) to obtain a plurality of traces. Each trace in the corresponding plurality of traces corresponds to an abundance measurement of a corresponding substance, which are over time collectively determined from the corresponding plurality of dynamic biological response samples.

Next, a respective second dataset may be obtained through the application of recurrence quantification analysis (RQA) or related methods to the corresponding plurality of traces in order to measure a corresponding set of features, each respective feature in the corresponding set of features being determined by a variation of abundance of one or more substances in the corresponding plurality of traces.

Next, an untrained or partially untrained model may be generated, with (i) the corresponding set of features of each respective second dataset of each training subject in the plurality of training subjects and (ii) the corresponding diagnostic status of each training subject in the plurality of training subjects, selected from among the first diagnostic status and the second diagnostic status, thereby obtaining a trained model. The trained model provides an indication as to whether a test subject has the first biological condition based on values for features in a set of features acquired from a biological sample of the test subject. In some embodiments, the trained model is a neural network algorithm, a convolutional neural network algorithm, a support vector machine algorithm, a decision tree algorithm, an unsupervised clustering model algorithm, a supervised clustering model algorithm, a regression model, or any combination or variant thereof, particularly including gradient-boosting implementations of the described algorithms, e.g. gradient-boosted decision trees. In some embodiments, the trained model predicts outcomes relative to a multinomial or binomial distribution. In some embodiments, the trained model can be used to make a binary prediction as to whether a sample was derived from a subject with the first biological condition or not; or, may be multinomial, distinguishing subjects with no diagnosis from those with the first biological condition or a second biological condition, where the second biological condition is distinct from the first biological condition.

In some embodiments, the model is a neural network or a convolutional neural network. See, Vincent et al., 2010, "Stacked denoising autoencoders: Learning useful representations in a deep network with a local denoising criterion," J Mach Learn Res 11, pp. 3371-3408; Larochelle et al., 2009, "Exploring strategies for training deep neural networks," J Mach Learn Res 10, pp. 1-40; and Hassoun, 1995, Fundamentals of Artificial Neural Networks, Massachusetts Institute of Technology.

SVMs are described in Cristianini and Shawe-Taylor, 2000, "An Introduction to Support Vector Machines," Cambridge University Press, Cambridge; Boser et al., 1992, "A training algorithm for optimal margin classifiers," in Proceedings of the 5th Annual ACM Workshop on Computational Learning Theory, ACM Press, Pittsburgh, Pa., pp. 142-152; Vapnik, 1998, Statistical Learning Theory, Wiley, New York; Mount, 2001, Bioinformatics: sequence and genome analysis, Cold Spring Harbor Laboratory Press, Cold Spring Harbor, N.Y.; Duda, Pattern Classification, Second Edition, 2001, John Wiley & Sons, Inc., pp. 259, 262-265; and Hastie, 2001, The Elements of Statistical Learning, Springer, New York; and Furey et al., 2000, Bioinformatics 16, 906-914. When used for classification, SVMs separate a given set of binary labeled data with a hyper-plane that is maximally distant from the labeled data. For cases in which no linear separation is possible, SVMs can work in combination with the technique of 'kernels', which automatically realizes a non-linear mapping to a feature space. The hyper-plane found by the SVM in feature space corresponds to a non-linear decision boundary in the input space.

Decision trees are described generally by Duda, 2001, Pattern Classification, John Wiley & Sons, Inc., New York, pp. 395-396. Tree-based methods partition the feature space into a set of rectangles, and then fit a model (like a constant) in each one. In some embodiments, the decision tree is random forest regression. One specific algorithm that can be used is a classification and regression tree (CART). Other specific decision tree algorithms include, but are not limited to, ID3, C4.5, MART, and Random Forests. CART, ID3, and C4.5 are described in Duda, 2001, Pattern Classification, John Wiley & Sons, Inc., New York. pp. 396-408 and pp. 411-412. CART, MART, and C4.5 are described in Hastie et al., 2001, The Elements of Statistical Learning, Springer-Verlag, New York, Chapter 9. Random Forests are described in Breiman, 1999, "Random Forests-Random Features," Technical Report 567, Statistics Department, U.C. Berkeley, September 1999.

Clustering (e.g., unsupervised clustering model algorithms and supervised clustering model algorithms) is described at pages 211-256 of Duda and Hart, Pattern Classification and Scene Analysis, 1973, John Wiley & Sons, Inc., New York, (hereinafter "Duda 1973"). As described in Section 6.7 of Duda 1973, the clustering problem is described as one of finding natural groupings in a dataset. To identify natural groupings, two issues are addressed. First, a way to measure similarity (or dissimilarity) between two samples is determined. This metric (similarity measure) is used to ensure that the samples in one cluster are more like one another than they are to samples in other clusters. Second, a mechanism for partitioning the data into clusters using the similarity measure is determined. Similarity measures are discussed in Section 6.7 of Duda 1973, where it is stated that one way to begin a clustering investigation is to define a distance function and to compute the matrix of distances between all pairs of samples in the training set. If distance is a good measure of similarity, then the distance between reference entities in the same cluster will be significantly less than the distance between the reference entities in different clusters. However, as stated on page 215 of Duda 1973, clustering does not require the use of a distance metric. For example, a nonmetric similarity function $s(x, x')$ can be used to compare two vectors $x$ and $x'$. Conventionally, $s(x, x')$ is a symmetric function whose value is large when $x$ and $x'$ are somehow "similar." An example of a nonmetric similarity function $s(x, x')$ is provided on page 218 of Duda 1973. Once a method for measuring "similarity" or "dissimilarity" between points in a dataset has been selected, clustering requires a criterion function that measures the clustering quality of any partition of the data. Partitions of the data set that extremize the criterion function are used to cluster the data. See page 217 of Duda 1973. Criterion functions are discussed in Section 6.8 of Duda 1973. More recently, Duda et al., Pattern Classification, 2nd edition, John Wiley & Sons, Inc. New York, has been published. Pages 537-563 describe clustering in detail. More information on clustering techniques can be found in Kaufman and Rousseeuw, 1990, Finding Groups in Data: An Introduction to Cluster Analysis, Wiley, New York, N.Y.; Everitt, 1993, Cluster analysis (3d ed.), Wiley, New York, N.Y.; and Backer, 1995, Computer-Assisted Reasoning in Cluster Analysis, Prentice Hall, Upper Saddle River, New Jersey. Particular exemplary clustering techniques that can be used in the present disclosure include, but are not limited to, hierarchical clustering (agglomerative clustering using nearest-neighbor algorithm, farthest-neighbor algorithm, the average linkage algorithm, the centroid algorithm, or the sum-of-squares algorithm), k-means clustering, fuzzy k-means clustering algorithm, and Jarvis-Patrick clustering. In some embodiments, the clustering comprises unsupervised clustering, where no preconceived notion of what clusters should form when the training set is clustered, are imposed.

Regression models, such as that of the multi-category logit models, are described in Agresti, An Introduction to Categorical Data Analysis, 1996, John Wiley & Sons, Inc., New York, Chapter 8. In some embodiments, the model makes use of a regressor disclosed in Hastie et al., 2001, The Elements of Statistical Learning, Springer-Verlag, New York. In some embodiments, gradient-boosting models are used toward, for example, the classification algorithms described herein; these gradient-boosting models are described in Boehmke, Bradley; Greenwell, Brandon (2019). "Gradient Boosting". Hands-On Machine Learning with R. Chapman & Hall. pp. 221-245. ISBN 978-1-138-49568-5. In some embodiments, ensemble modeling techniques are used, for example, toward the classification algorithms described herein; these ensemble modeling techniques are described in the implementation of classification models herein, are described in Zhou Zhihua (2012). Ensemble Methods: Foundations and Algorithms. Chapman and Hall/CRC. ISBN 978-1-439-83003-1.

The model may be performed by a device executing one or more programs (e.g., one or more programs stored in the Non-Persistent Memory 111 or in the Persistent Memory 112 in Figure 1) including instructions to perform the data analysis. The data analysis may be performed by a system comprising at least one processor (e.g., the processing core 102) and memory (e.g., one or more programs stored in the Non-Persistent Memory 111 or in the Persistent Memory 112) comprising instructions to perform the data analysis.

## Computer systems

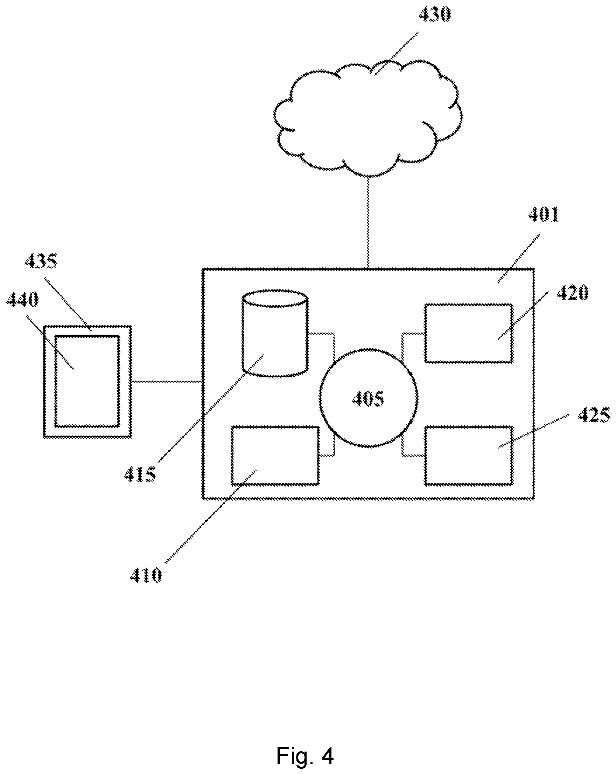

Disclosed but not claimed are computer systems that are programmed to implement the methods of the disclosure. **FIG. 4** shows a computer system 401 that is programmed or otherwise configured to, for example, stain a tooth sample, obtain a fluorescence image of stained tooth samples, analyze a fluorescence intensity spatially across stained tooth samples, generate a temporal profile of inflammation, process data using trained models, and determine a risk of a disease or disorder of a subject. The computer system 401 can regulate various aspects of sensor data analysis of the present disclosure, such as, for example, staining a tooth sample, obtaining a fluorescence image of stained tooth samples, analyzing a fluorescence intensity spatially across stained tooth samples, generating a temporal profile of inflammation, measuring the dynamics of the temporal profile, process data using trained models, and predicting a subject's diagnostic status with respect to a disease or disorder. The computer system 401 can be an electronic device of a user or a computer system that is remotely located with respect to the electronic device. The electronic device can be a mobile electronic device.

The computer system 401 includes a central processing unit (CPU, also "processor" and "computer processor" herein) 405, which can be a single core or multi core processor, or a plurality of processors for parallel processing. The computer system 401 also includes memory or memory location 410 (e.g., random-access memory, read-only memory, flash memory), electronic storage unit 415 (e.g., hard disk), communication interface 420 (e.g., network adapter) for communicating with one or more other systems, and peripheral devices 425, such as cache, other memory, data storage and/or electronic display adapters. The memory 410, storage unit 415, interface 420 and peripheral devices 425 are in communication with the CPU 405 through a communication bus (solid lines), such as a motherboard. The storage unit 415 can be a data storage unit (or data repository) for storing data. The computer system 401 can be operatively coupled to a computer network ("network") 430 with the aid of the communication interface 420. The network 430 can be the Internet, an internet and/or extranet, or an intranet and/or extranet that is in communication with the Internet. The network 430 in some cases is a telecommunication and/or data network. The network 430 can include one or more computer servers, which can enable distributed computing, such as cloud computing. The network 430, in some cases with the aid of the computer system 401, can implement a peer-to-peer network, which may enable devices coupled to the computer system 401 to behave as a client or a server.

The CPU 405 can execute a sequence of machine-readable instructions, which can be embodied in a program or software. The instructions may be stored in a memory location, such as the memory 410. The instructions can be directed to the CPU 405, which can subsequently program or otherwise configure the CPU 405 to implement methods of the present disclosure. Examples of operations performed by the CPU 405 can include fetch, decode, execute, and writeback.

The CPU 405 can be part of a circuit, such as an integrated circuit. One or more other components of the system 401 can be included in the circuit. In some cases, the circuit is an application specific integrated circuit (ASIC).

The storage unit 415 can store files, such as drivers, libraries and saved programs. The storage unit 415 can store user data, e.g., user preferences and user programs. The computer system 401 in some cases can include one or more additional data storage units that are external to the computer system 401, such as located on a remote server that is in communication with the computer system 401 through an intranet or the Internet.

The computer system 401 can communicate with one or more remote computer systems through the network 430. For instance, the computer system 401 can communicate with a remote computer system of a user (e.g., a health care provider). Examples of remote computer systems include personal computers (e.g., portable PC), slate or tablet PC's (e.g., Apple® iPad, Samsung® Galaxy Tab), telephones, Smart phones (e.g., Apple® iPhone, Android-enabled device, Blackberry®), or personal digital assistants. The user can access the computer system 401 via the network 430.

Methods as described herein can be implemented by way of machine (e.g., computer processor) executable code stored on an electronic storage location of the computer system 401, such as, for example, on the memory 410 or electronic storage unit 415. The machine executable or machine-readable code can be provided in the form of software. During use, the code can be executed by the processor 405. In some cases, the code can be retrieved from the storage unit 415 and stored on the memory 410 for ready access by the processor 405. In some situations, the electronic storage unit 415 can be precluded, and machine-executable instructions are stored on memory 410.

The code can be pre-compiled and configured for use with a machine having a processer adapted to execute the code, or can be compiled during runtime. The code can be supplied in a programming language that can be selected to enable the code to execute in a pre-compiled or as-compiled fashion.

Aspects of the systems and methods provided herein, such as the computer system 401, can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such as memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system 401 can include or be in communication with an electronic display 435 that comprises a user interface (UI) 440 for providing, for example, fluorescence image data, fluorescence intensity data, temporal profiles of inflammation, and machine learning classifications. Examples of UI's include, without limitation, a graphical user interface (GUI) and web-based user interface.

Methods and systems of the present disclosure can be implemented by way of one or more algorithms. An algorithm can be implemented by way of software upon execution by the central processing unit 405. The algorithm can, for example, stain a tooth sample, obtain a fluorescence image of stained tooth samples, analyze a fluorescence intensity spatially across stained tooth samples, generate a temporal profile of inflammation, process data using trained models, and determine a risk of a disease or disorder of a subject

One or more of the steps of each of the methods or sets of operations may be performed with circuitry as described herein, for example, one or more of the processor or logic circuitry such as programmable array logic for a field programmable gate array. The circuitry may be programmed to provide one or more of the steps of each of the methods or sets of operations, and the program may comprise program instructions stored on a computer readable memory or programmed steps of the logic circuitry such as the programmable array logic or the field programmable gate array, for example.

**EXAMPLES**

**Example 1: Dynamic molecular profiles in tooth samples for determining disease risk**

Using methods and systems of the present disclosure, molecular profiles in tooth samples were generated and subsequently analyzed to determine a disease risk in a subject. Generally, the temporal dynamics of biological response (e.g., inflammation) were found to be imprinted in samples (e.g., tooth samples), and can be analyzed to determine disease risk in a subject. Dynamic molecular profiles were generated for C-reactive protein (CRP), which is a marker of inflammation. Using the tooth biomarkers, dynamic time-series profiles of CRP and inflammation were generated during a time period that comprised fetal (prenatal) development and early childhood in two sets of children-a first set with autism spectrum disorder (37 cases) and a second set without autism spectrum disorder (77 controls). The time-series CRP profiles were analyzed to reveal novel features of the dynamics of the CRP signal, which accurately distinguished the autism cases from controls. For example, the inflammation profiles that were present before age of 1 year were highly differential between cases and controls. In comparison, a clinical diagnosis of autism is usually determined around the age of 3 to 4 years.

A primary tooth sample was obtained from each child subject. The teeth samples were sectioned open, decalcified and an immunohistochemistry stain (e.g., dentine) was applied to the teeth samples. The immunohistochemistry stain effectively mapped C-reactive protein (a molecular marker of inflammation) along the growth rings of the teeth samples in order to develop temporal profiles of inflammation over the prenatal and postnatal period. The temporal profiles were analyzed using machine learning algorithms of the present disclosure to train highly accurate models to determine disease risk (e.g., autism).

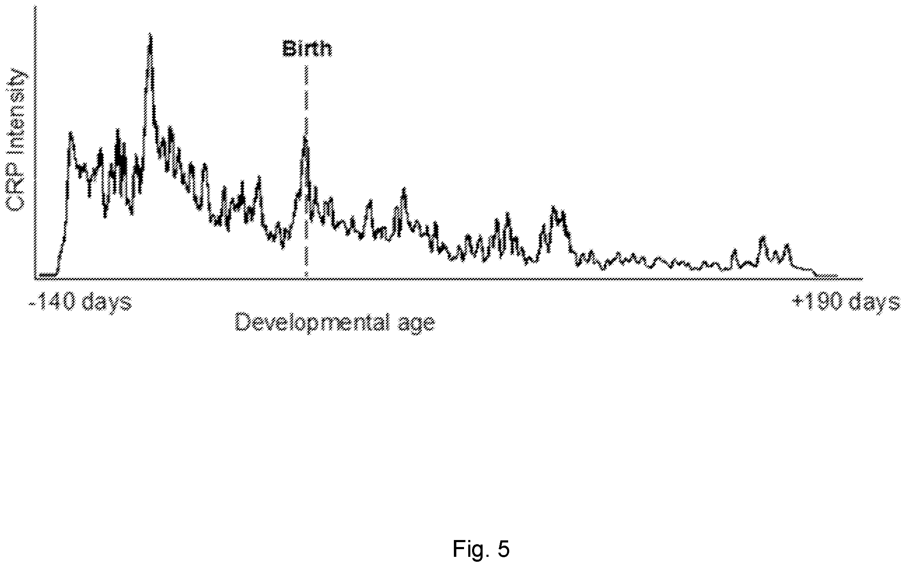

FIG. 5 shows an example of a daily C-reactive protein profile of a subject over time, where the y-axis is indicative of CRP intensity and the x-axis is indicative of developmental age. The developmental age of the child subject included a time period ranging from the second trimester of gestation (e.g., starting at 140 days before birth, when the subject was in the prenatal stage) to about 6 months of age. As shown in FIG. 5, inflammation (as indicated by CRP intensity) profiles in cases of children with autism were observed to be higher prenatally.

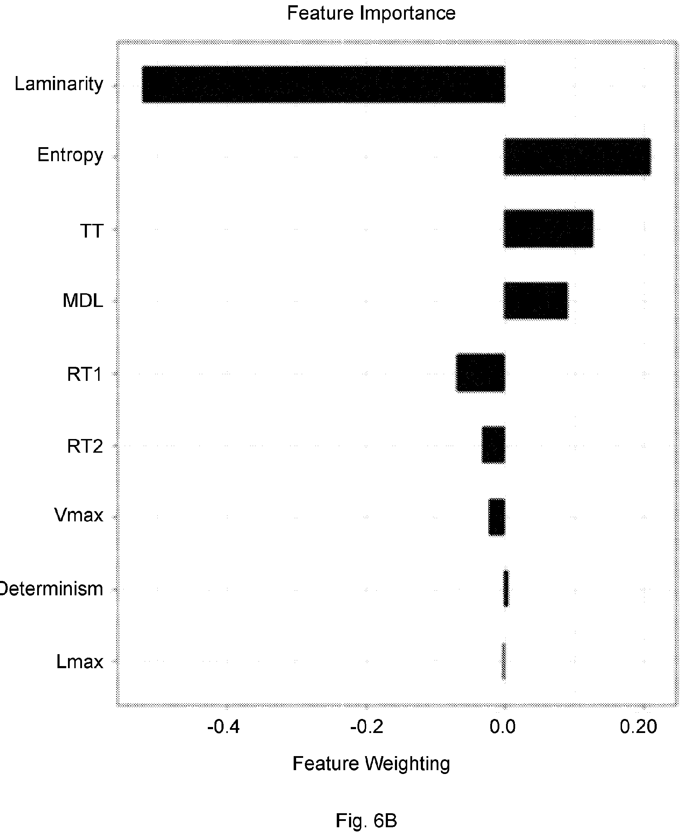

FIG. 6A-6B show a receiver operating characteristic (ROC) curve to characterize the sensitivity and specificity of the method for diagnosing autism at varying predictive thresholds utilizing features derived from recurrence quantification analysis of C-reactive protein profiles sampled prenatally and in early childhood (e.g., up to 1 year of age). FIG. 6A shows an experimental Receiver Operating Characteristics (ROC) curve for evaluating accuracy of the disclosed method of evaluating a subject for autism spectrum disorder. A ROC curve can be used for evaluating a performance of a binary classifier. A ROC curve is plotted as sensitivity (also called as a true positive rate) against specificity (also called as a true negative rate). A perfect classifier may have a 100% sensitivity and 100% specificity and an Area-Under-the-Curve (AUC) of 1.0. As shown in FIG. 6A, the classifier configured to determine the presence of autism in a subject based on dynamic C-reactive protein profile had an Area-Under-the-Curve (AUC) of the receiver operating characteristic (ROC) of 0.86, with a 95% confidence interval (CI) of 0.72 to 1.00. The receiver operating characteristic (ROC) shows how sensitivity and specificity values of the classifier change as higher or lower thresholds are applied to predicted probabilities of case status; a lower threshold will yield a more sensitive classification, for example, but will be correspondingly less specific. As shown in FIG. 6B, the primary dynamical features which contribute to classifier performance, which are ranked in descending order of feature importance (e.g., as indicated by the numerical feature weighting) include laminarity, entropy, TT, MDL, RT1, RT2, Vmax, Determinism, and Lmax. For example, laminarity was determined to have higher feature importance than the others.

Therefore, analysis of features derived from analysis of C-reactive protein profiles using methods and systems of the present disclosure successfully determined the disease risk of autism with an AUC of 0.86, using only features derived from analysis of C-reactive protein signatures measured on non-invasively obtained biological samples (e.g., tooth samples) from child subjects. These results demonstrate that dynamics of inflammatory response in early life are linked to disease later on, which can be accurately detected and profiled using methods and systems of the present disclosure.

## Claims

1. A method for predicting a subject's diagnostic status with respect to a disease or disorder, comprising:

(a) obtaining by imaging a fluorescence image of a stained tooth sample of the subject, wherein the stained tooth sample is stained using an immunohistochemistry stain;
(b) analyzing the stain fluorescence intensity of the fluorescence image spatially across the stained tooth sample; and
(c) predicting the subject's diagnostic status with respect to the disease or disorder based at least in part on the analysis of the fluorescence intensity, wherein the disease or disorder comprises autism spectrum disorder (ASD);

wherein staining the tooth sample comprises using a C-reactive protein immunohistochemistry stain; and
wherein the subject is is human and less than 12 years old.

2. The method of claim 1, wherein obtaining the fluorescence image of the stained tooth sample comprises using an inverted or non-inverted confocal microscope.

3. .The method of claim 1 or 2, further comprising sectioning the tooth sample.

4. The method of any one of claims 1-3, wherein staining the tooth sample comprises decalcifying the tooth sample.

5. The method of any one of claims 1-4, wherein the subject is less than 1 year old.

6. The method of any one of claims 1-5, wherein the analyzing comprises generating a temporal profile of inflammation based at least in part on the fluorescence intensity, and analyzing the temporal profile of inflammation.

7. The method of claim 6, wherein at least a portion of the temporal profile of inflammation corresponds to a prenatal period of the subject.

8. The method of any one of claims 1-7, wherein predicting the subject's diagnostic status with respect to the disease or disorder comprises processing the fluorescence intensity using a trained model.

9. The method of claim 8, wherein the trained model is selected from the group consisting of: a neural network algorithm, a support vector machine algorithm, a decision tree algorithm, an unsupervised clustering algorithm, a supervised clustering algorithm, a regression algorithm, a gradient-boosting algorithm, and any combination thereof, or
wherein the trained model comprises a gradient-boosted decision tree.

10. The method of claim 9, wherein the trained model is configured to process one or more features selected from the group consisting of recurrence rates, determinism, mean diagonal length, maximum diagonal length, divergence, Shannon entropy in diagonal length, trend in recurrences, laminarity, trapping time, maximum vertical line length, Shannon entropy in vertical line lengths, mean recurrence time, Shannon entropy in recurrence times, number of the most probable recurrences, mean diagonal length (MDL), recurrence time (RT), Vmax, determinism, Lmax, and any combination thereof.

11. The method of claim 10, wherein the trained model is configured to process two or more features selected from the group consisting of recurrence rates, determinism, mean diagonal length, maximum diagonal length, divergence, Shannon entropy in diagonal length, trend in recurrences, laminarity, trapping time (TT), maximum vertical line length, Shannon entropy in vertical line lengths, mean recurrence time, Shannon entropy in recurrence times, number of the most probable recurrences, mean diagonal length (MDL), recurrence time (RT), Vmax, determinism, Lmax, and any combination thereof.

12. The method of claim 11, wherein the trained model predicts the subject's diagnostic status with respect to the disease or disorder with a sensitivity of at least about 80%, or

> wherein the trained model predicts the subject's diagnostic status with respect to the disease or disorder with a specificity of at least about 80%, or
> wherein the trained model predicts the subject's diagnostic status with respect to the disease or disorder with a positive predictive value of at least about 80%, or
> wherein the trained model predicts the subject's diagnostic status with respect to the disease or disorder with a negative predictive value of at least about 80%, or
> wherein the trained model predicts the subject's diagnostic status with respect to the disease or disorder with an Area Under the Receiver Operating Characteristic (AUROC) of at least about 0.80.

**Patentansprüche**

1. Ein Verfahren zum Vorhersagen des Diagnosestatus eines Subjekts in Bezug auf eine Erkrankung oder Störung, umfassend:

> a) Erhalten einer Fluoreszenzaufnahme von einer gefärbten Zahnprobe des Subjekts durch Aufnahme, wobei die gefärbte Zahnprobe durch eine Immunhistochemie-Färbung gefärbt wurde;
> b) Analysieren der Fluoreszenzintensität der Fluoreszenzaufnahme über die gesamte Fläche der gefärbten Zahnprobe;
> c) Vorhersagen des mindestens teilweise auf der Analyse der Fluoreszenzintensität basierenden Diagnosestatus des Subjekts in Bezug auf die Erkrankung oder Störung, wobei die Erkrankung oder Störung Autismus-Spektrum-Störung (ASD) umfasst;

> wobei die Färbung der Zahnprobe die Verwendung eines C-reaktiven Protein-Immunhistochemie-Stammes umfasst; und
> wobei das Subjekt ein Mensch und unter 12 Jahre alt ist.

2. Das Verfahren nach Anspruch 1, wobei das Erhalten der Fluoreszenzaufnahme von der gefärbten Zahnprobe die Verwendung eines invertierten oder nicht-invertierten Konfokal-Mikroskops umfasst.

3. Das Verfahren nach Anspruch 1 oder 2 ferner umfassend das Aufteilen der Zahnprobe.

4. Das Verfahren nach einem der Ansprüche 1 bis 3, wobei das Färben der Zahnprobe das Entkalken der Zahnprobe umfasst.

5. Das Verfahren nach einem der Ansprüche 1 bis 4, wobei das Subjekt unter einem Jahr alt ist.

6. Das Verfahren nach einem der Ansprüche 1 bis 5, wobei das Analysieren das Erzeugen eines mindestens teilweise auf der Fluoreszenzintensität basierenden temporären Entzündungsprofils umfasst, und Analysieren des temporären Entzündungsprofils.

7. Das Verfahren nach Anspruch 6, wobei mindestens ein Teil des temporären Entzündungsprofils zur pränatalen Phase des Subjekts gehört.

8. Das Verfahren nach einem der Ansprüche 1 bis 7, wobei das Vorhersagen des Diagnosestatus des Subjekts in Bezug auf die Erkrankung oder Störung das Prozessieren der Fluoreszenzintensität unter Verwendung eines trainierten Models umfasst.

9. Das Verfahren nach Anspruch 8, wobei das trainierte Model ausgewählt ist aus der Gruppe bestehend aus: ein neuronaler Netzwerkalgorithmus, ein Support-Vektor-Maschinenalgorithmus, ein Entscheidungsbaumalgorithmus, ein unüberwachter Clustering-Algorithmus, ein überwachter Clustering-Algorithmus, ein Regressionsalgorithmus, ein Gradientenverstärkungsalgorithmus und jede Kombination davon, oder wobei das trainierte Modell einen gradientenverstärkten Entscheidungsbaum umfasst.

10. Das Verfahren nach Anspruch 9, wobei das trainierte Model konfiguriert ist, um eine oder mehrere Funktionen ausgewählt aus der Gruppe bestehend aus Wiederholungsraten, Determinismus, mittlere Diagonallänge, maximale Diagonallänge, Divergenz, Shannon-Entropie in der Diagonallänge, Trend bei Wiederholungen, Laminarität, Verweildauer, maximale vertikale Linienlänge, Shannon-Entropie in vertikalen Linienlängen, mittlere Wiederholungszeit, Shannon-Entropie in Wiederholungszeiten, Anzahl der wahrscheinlichsten Wiederholungen, mittlere Diagonallänge (MDL), Wiederholungszeit (RT), Vmax, Determinismus, Lmax und jede Kombination davon zu verarbeiten.

11. Das Verfahren nach Anspruch 10, wobei das trainierte Model konfiguriert ist, um zwei oder mehrere Funktionen ausgewählt aus der Gruppe bestehend aus Wiederholungsraten, Determinismus, mittlere Diagonallänge, maximale Diagonallänge, Divergenz, Shannon-Entropie in der Diagonallänge, Trend bei Wiederholungen, Laminarität, Verweildauer, maximale vertikale Linienlänge, Shannon-Entropie in vertikalen Linienlängen, mittlere Wiederholungszeit, Shannon-Entropie in Wiederholungszeiten, Anzahl der wahrscheinlichsten Wiederholungen, mittlere Diagonallänge (MDL), Wiederholungszeit (RT), Vmax, Determinismus, Lmax und jede Kombination davon zu verarbeiten.

12. Das Verfahren nach Anspruch 11, wobei das trainierte Model den Diagnosestatus des Subjekts in Bezug auf die Erkrankung oder Störung mit einer Empfindlichkeit von mindestens etwa 80% vorhersagt, oder

wobei das trainierte Model den Diagnosestatus des Subjekts in Bezug auf die Erkrankung oder Störung mit einer Spezifität von mindestens etwa 80% vorhersagt, oder
wobei das trainierte Model den Diagnosestatus des Subjekts in Bezug auf die Erkrankung oder Störung mit einem positiven Vorhersagewert von mindestens etwa 80% vorhersagt, oder
wobei das trainierte Model den Diagnosestatus des Subjekts in Bezug auf die Erkrankung oder Störung mit einem negativen Vorhersagewert von mindestens etwa 80% vorhersagt, oder
wobei das trainierte Model den Diagnosestatus des Subjekts in Bezug auf die Erkrankung oder Störung mit einer Fläche unter der Grenzwertoptimierungskurve (AUROC) von mindestens etwa 0,80 vorhersagt.

**Revendications**

1. Un procédé pour prédire un état diagnostique d'un sujet par rapport à une maladie ou un trouble, comprenant :

(a) l'obtention par imagerie d'une image par fluorescence d'un échantillon de dent coloré du sujet, dans lequel l'échantillon de dent coloré est coloré à l'aide d'un colorant immunohistochimique ;
(b) l'analyse de l'intensité de fluorescence de colorant de l'image de fluorescence spatialement à travers l'échantillon de dent coloré ; et
(c) la prédiction de l'état diagnostique du sujet par rapport à la maladie ou au trouble sur la base au moins en partie de l'analyse de l'intensité de fluorescence, dans lequel la maladie ou le trouble comprend le trouble du spectre autistique (TSA) ;

dans lequel la coloration de l'échantillon de dent comprend l'utilisation d'un colorant immunohistochimique de protéine C-réactive ; et
dans lequel le sujet est humain et âgé de moins de 12 ans.

2. Le procédé selon la revendication 1, dans lequel l'obtention de l'image de fluorescence de l'échantillon de dent coloré comprend l'utilisation d'un microscope confocal inversé ou non inversé.

3. Le procédé selon la revendication 1 ou 2, comprenant en outre le sectionnement de l'échantillon de dent.

4. Le procédé selon l'une quelconque des revendications 1-3, dans lequel la coloration de l'échantillon de dent comprend le détartrage de l'échantillon de dent.

5. Le procédé selon l'une quelconque des revendications 1-4, dans lequel le sujet a moins d'un an.

6. Le procédé selon l'une quelconque des revendications 1-5, dans lequel l'analyse comprend la génération d'un profil temporel d'inflammation sur la base au moins en partie de l'intensité de fluorescence, et l'analyse du profil temporel d'inflammation.

7. Le procédé selon la revendication 6, dans lequel au moins une partie du profil temporel d'inflammation correspond à une période prénatale du sujet.

8. Le procédé selon l'une quelconque des revendications 1-7, dans lequel la prédiction de l'état diagnostique du sujet par rapport à la maladie ou au trouble comprend le traitement de l'intensité de fluorescence à l'aide d'un modèle entraîné.

9. Le procédé selon la revendication 8, dans lequel le modèle entraîné est sélectionné dans le groupe consistant en : un algorithme de réseau neuronal, un algorithme de machine de vecteur de support, un algorithme d'arbre de décision, un algorithme de clustering non supervisé, un algorithme de clustering supervisé, un algorithme de régression, un algorithme de gradient boosting, et toute combinaison de ceux-ci, ou
dans lequel le modèle entraîné comprend un arbre de décision boosté par gradient.

10. Le procédé selon la revendication 9, dans lequel le modèle entraîné est configuré pour traiter une ou plusieurs caractéristiques sélectionnées dans le groupe consistant en taux de récurrence, déterminisme, longueur diagonale moyenne, longueur diagonale maximale, divergence, entropie de Shannon en longueur diagonale, tendance en récurrences, laminarité, temps de piégeage, longueur de ligne verticale maximale, entropie de Shannon en longueurs de ligne verticales, temps de récurrence moyen, entropie de Shannon en temps de récurrence, nombre de récurrences les plus probables, longueur diagonale moyenne (MDL), temps de récurrence (RT), Vmax, déterminisme, Lmax et toute combinaison de ceux-ci.

11. Le procédé selon la revendication 10, dans lequel le modèle entraîné est configuré pour traiter deux caractéristiques ou plus sélectionnées dans le groupe consistant en taux de récurrence, déterminisme, longueur diagonale moyenne, longueur diagonale maximale, divergence, entropie de Shannon en longueur diagonale, tendance en récurrences, laminarité, temps de piégeage (TT), longueur de ligne verticale maximale, entropie de Shannon en longueurs de ligne verticales, temps de récurrence moyen, entropie de Shannon en temps de récurrence, nombre de récurrences les plus probables, longueur diagonale moyenne (MDL), temps de récurrence (RT), Vmax, déterminisme, Lmax et toute combinaison de ceux-ci.

12. Le procédé selon la revendication 11, dans lequel le modèle entraîné prédit l'état diagnostique du sujet par rapport à la maladie ou au trouble avec une sensibilité d'au moins environ 80 %, ou

dans lequel le modèle entraîné prédit l'état diagnostique du sujet par rapport à la maladie ou au trouble avec une spécificité d'au moins environ 80 %, ou
dans lequel le modèle entraîné prédit l'état diagnostique du sujet par rapport à la maladie ou au trouble avec une valeur prédictive positive d'au moins environ 80 %, ou
dans lequel le modèle entraîné prédit l'état diagnostique du sujet par rapport à la maladie ou au trouble avec une valeur prédictive négative d'au moins environ 80 %, ou
dans lequel le modèle entraîné prédit l'état diagnostique du sujet par rapport à la maladie ou au trouble avec une aire sous la caractéristique de fonctionnement du récepteur (AUROC) d'au moins environ 0,80.

System 100

Non-Persistent
Memory 111

| | |
|---|---|
| Operating system | 116 |
| Network communication module | 118 |
| Classifier training module | 120 |
| Dataset for biological samples from training subjects | 122 |
| Training subject 1 | 124-1 |
| Feature 1 | 126-1-1 |
| Feature 2 | 126-1-2 |
| • • • | |
| Feature M | 126-1-M |
| Diagnostic status | 128-1 |
| Training subject 2 | 124-2 |
| Feature 1 | 126-2-1 |
| Feature 2 | 126-2-2 |
| • • • | |
| Feature M | 126-2-M |
| Diagnostic status | 128-2 |
| • • • | |
| Training subject Y | 124-Y |
| Classifier validation module | 130 |
| Dataset for biological samples from validation subjects | 132 |
| Patient classification module | 134 |
| • • • | |

Processing
core          102

114

104

Network
interface

106

User interface

Display          108

Input

110

Persistent memory 112

Fig. 1

EP 4 256 345 B1

201

200B

200A

100 μm

Fig. 2A

Fig. 2B

Fig. 2C

300 ─

```
┌─────────────────────────────────────────────┐
│  Stain a tooth sample of the subject to produce a  │
│            stained tooth sample             │ ──── 302
└─────────────────────────────────────────────┘
                        │
                        ▼
┌─────────────────────────────────────────────┐
│  Analyze a fluorescence intensity spatially across  │
│             the stained tooth sample             │ ──── 304
└─────────────────────────────────────────────┘
                        │
                        ▼
┌─────────────────────────────────────────────┐
│  Determine the risk of the disease or disorder of  │
│  the subject based at least in part on the analysis  │
│            of the fluorescence intensity            │ ──── 306
└─────────────────────────────────────────────┘
```

Fig. 3

Fig. 4

Fig. 5

Fig. 6A

Feature Importance

Fig. 6B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **AUSTIN C. et al.** *SCIENTIFIC REPORTS*, 2016, vol. 6, 18802
- **MORISHITA H. et al.** *TRENDS IN NEUROSCIENCES*, 2016, vol. 40 (1), 1-3
- **WEBBER et al.** Simpler Methods Do It Better: Success of Recurrence Quantification Analysis as a General Purpose Data Analysis Tool. *Physics Letters A*, 2009, vol. 373, 3753-3756
- **MARWAN et al.** Recurrence Plots for the Analysis of Complex Systems. *Physics Reports*, 2007, vol. 438, 237-239
- **VINCENT et al.** Stacked denoising autoencoders: Learning useful representations in a deep network with a local denoising criterion. *J Mach Learn Res*, 2010, vol. 11, 3371-3408
- **LAROCHELLE et al.** Exploring strategies for training deep neural networks. *J Mach Learn Res*, 2009, vol. 10, 1-40
- **HASSOUN**. Fundamentals of Artificial Neural Networks. Massachusetts Institute of Technology, 1995
- **CRISTIANINI** ; **SHAWE-TAYLOR**. An Introduction to Support Vector Machines. Cambridge University Press, 2000
- A training algorithm for optimal margin classifiers. **BOSER et al.** Proceedings of the 5th Annual ACM Workshop on Computational Learning Theory. ACM Press, 1992, 142-152
- **VAPNIK**. Statistical Learning Theory. Wiley, 1998
- **MOUNT**. Bioinformatics: sequence and genome analysis. Cold Spring Harbor Laboratory Press, 2001
- **DUDA**. Pattern Classification. John Wiley & Sons, Inc., 2001, vol. 259, 262-265
- **HASTIE**. The Elements of Statistical Learning. Springer, 2001
- **FUREY et al.** *Bioinformatics*, 2000, vol. 16, 906-914
- **DUDA**. Pattern Classification. John Wiley & Sons, Inc., 2001, 395-396
- **DUDA**. Pattern Classification. John Wiley & Sons, Inc., 2001, 396-408, 411-412
- **HASTIE et al.** The Elements of Statistical Learning. Springer-Verlag, 2001
- Random Forests-Random Features. **BREIMAN** ; **U.C. BERKELEY**. Technical Report. Statistics Department, September 1999, 567
- **DUDA** ; **HART**. Pattern Classification and Scene Analysis. John Wiley & Sons, Inc., 1973, 211-256
- **DUDA et al.** Pattern Classification. John Wiley & Sons, Inc., 537-563
- **KAUFMAN** ; **ROUSSEEUW**. Finding Groups in Data: An Introduction to Cluster Analysis. Wiley, 1990
- **EVERITT**. Cluster analysis. Wiley, 1993
- **BACKER**. Computer-Assisted Reasoning in Cluster Analysis. *Prentice Hall*, 1995
- **AGRESTI**. An Introduction to Categorical Data Analysis. John Wiley & Sons, Inc., 1996
- **BOEHMKE** ; **BRADLEY** ; **GREENWELL** ; **BRANDON**. Gradient Boosting. *Hands-On Machine Learning with R. Chapman & Hall.*, 2019, 221-245
- **ZHOU ZHIHUA**. Ensemble Methods: Foundations and Algorithms. Chapman and Hall/CRC, 2012